# EUROPEAN PATENT APPLICATION

(11) **EP 1 975 178 A1**
(43) Date of publication of application: **01.10.2008**
(21) Application number: 07450065.3
(22) Date of filing: 30.03.2007
(51) Int. Cl.: C07K 16/00

(54) **Transcytotic modular antibody**

(71) Applicant: f-star Biotechnologische Forschungs- und Entwicklungsges.m.b.H., 1230 Wien (AT)
(72) Inventor: Himmler, Gottfried, 2301 Gross-Enzersdorf (AT); Redl, Gerda, 2301 Gross-Enzersdorf (AT)
(74) Representative: Schwarz, Albin

(57) **Abstract**

The present application relates to a transcytotic modular antibody containing a de novo pH dependent binding site for a transport protein, a method of producing such a modular antibody by engineering a molecule containing an antibody domain to modify a structural loop, libraries containing such modular antibodies, and a method of prolonging the half-life of modular antibodies and biologically active molecules.

SEQUENCE LISTING

<110> f-star Biotechnologische Forschungs- und
      Entwicklungsges.m.b.H.

<120> Transcytotic modular antibody

<130> FS7/EP

<140> EP07450065
<141> 2007-03-30

<160> 1

<170> PatentIn version 3.3

<210> 1
<211> 232
<212> PRT
<213> human

<400> 1

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr

            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

## Description

The invention refers to a transcytotic modular antibody having a binding site for a transport protein.

Transcytosis is the process by which various macromolecules are transported across the interior of a cell. For the transcellular transport a cell encloses extracellular material in an invagination of the cell membrane to form a vesicle, then moves the vesicle across the cell to eject the material through the opposite cell membrane by the reverse process. This is also called vesicular transport. While transcytosis is most commonly observed in cells containing an epithelium, the process is also present elsewhere. Blood capillaries are a well-known site for transcytosis, though it occurs in other cells, including neurons and intestinal cells.

The polymeric immunoglobulin receptor (pIgR) is transcytosed from the basolateral to the apical surface of polarized epithelial cells. Binding of a physiological ligand, dimeric IgA, to pIgR stimulates pIgR transcytosis.

IgG antibody transcytosis in epithelial cells is mediated by FcRn immunoglobulin receptor. The process of transporting IgG immunoglobulin, via transcytosis using the FcRn (also known as the neonatal Fc receptor; gene name FCGRT), from apical surface of an epithelial cell to the basolateral surface or vice versa is depending on the location. This process is used for uptake of IgG from the milk in the gut in rodents, for transplacental transport of IgG from mother to embryo in humans, and for maintenance of a steady-state distribution of IgG across epithelial boundaries in general in adult mammals.

In the kidney, proteins filtered through glomeruli are reabsorbed by endocytosis along the proximal tubules to avoid renal loss of large amounts of proteins. Indeed, various proteins such as albumin, b2- microglobulin and several hormones are reabsorbed via receptor-mediated endocytosis in the proximal tubules. Neonatal Fc receptor (FcRn), which is involved in the transport of IgG across several epithelial and endothelial cells, was also reported to be expressed in renal proximal tubular epithelial cells. It was suggested that FcRn may play an important role in the reabsorption of IgG from the tubular fluid. In a human IgG transport assay it revealed that receptor-mediated transepithelial transport of intact IgG in RPTECs is bidirectional and that it requires the formation of acidified intracellular compartments. It was suggested that the intact pathway for human IgG transepithelial transport may avoid lysosomal degradation of IgG. (Am J Physiol Renal Physiol 282: F358-F365, 2002).

The MHC class I-related receptor, FcRn, thus plays a central role in regulating the serum levels of IgG. FcRn is expressed in endothelial cells, suggesting that these cells may be involved in maintaining IgG levels. Segregation of FcRn-IgG complexes from unbound IgG occurs in the sorting endosome. FcRn or FcRn-IgG complexes are gradually depleted from sorting endosomes to ultimately generate multivesicular bodies whose contents are destined for lysosomal degradation. In addition, the pathways taken by FcRn and the transferrin receptor overlap, despite distinct mechanisms of ligand uptake. *(*The Journal of Immunology, 2004, 172: 2021-2029.)

There are two natural molecules of the body which have extraordinary long biological half lives - IgG and serum albumin (human serum albumin, HSA). The long half-life of these two molecules can be explained by binding to FcRn in a pH-dependent manner and consequent recycling into the vascular endothelium. These two proteins bind to FcRn in a non-competitive, yet similarly pH-dependent manner.

Proteins in the bloodstream, including IgG and HSA, enter the endothelial cells via fluid-phase endocytosis as the cells sample their environment (Berg et al., 1995). When IgG enters the endosomes of these endothelial cells, the neonatal Fc receptor, FcRn, binds to the Fc region of IgG (and to domain III in serum albumin). The binding of FcRn to Fc has been experimentally found to enhance the recycling of IgG and serum albumin back to the cell surface via a mechanism that is still not completely understood. This recycling decreases the amount of IgG and serum albumin that is degraded in the lysosomes. Dissociation of the Fc fragment or serum albumin from FcRn at the cell surface then releases IgG and HSA back into the bloodstream.

Fc and serum albumin have a greater binding affinity for FcRn in the endosome (pH 5-6) than at the cell surface (pH 7.4) because a favourable interaction between these molecules and FcRn is induced by the protonation of certain histidines of Fc at the lower pH (Ghetie and Ward, 2000).

This natural phenomenon of long half-life of certain proteins can be utilized to prolong in vivo half-life of therapeutics. Increasing the half-lives of therapeutics can decrease the frequency of patient treatments and may in addition allow administration of lower concentrations of the molecule, which can minimize the toxic side effects associated with delivering high concentrations of the therapeutic.

### Binding of therapeutic molecules to serum albumin or IgG

One possibility to prolong half-life is according to a "piggyback-strategy": The therapeutic molecule is bound either to immunoglobulin or serum albumin.

The disadvantage of this method is that the upper limit of half life is the natural half life of IgG or serum albumin. Another disadvantage is the critical affinity necessary to allow efficient recycling and to provide enough free therapeutic molecules in circulation.

### Fusion of therapeutic molecules to Protein A or Protein G

Fusion proteins containing the immunoglobulin-binding domains of staphylococcal protein A or the serum albumin-binding regions of streptococcal protein G (Stahl & Nygren, Pathol Biol (Paris). 1997 Jan;45(1):66-76) have been described as a possibilty to increase biological half life of a protein. However, these fusions also have been described to potentiate the denaturation and immunogenicity of the fusion partner molecule.

### Fusion or coupling of therapeutic molecules to serum albumin or Fc

Another strategy to prolong half life of biologically active compounds is the coupling of such compounds to Fc or serum albumin. Coupling can be performed by chemical means or by genetic fusion of protein chains.

A disadvantage of the method is the overall change in the therapeutic molecule structure and size.

### Structure of antibodies

The basic structure of antibodies is similar for various classes of antibodies and for various species and will be explained here using as example an intact IgG1 immunoglobulin.

Two identical heavy (H) and two identical light (L) chains combine to form the Y-shaped antibody molecule. The heavy chains each have four domains. Antibody domains are modular repeats with a conserved fold. The amino terminal variable domains (VH) are at the tips of the Y. These are followed by three constant domains: CH1, CH2, and the carboxy-terminal CH3, at the base of the Y's stem. A short stretch, the switch, connects the heavy chain variable and constant regions. The hinge connects CH2 and CH3 (the Fc fragment) to the remainder of the antibody (the Fab fragments). One Fc and two identical Fab fragments can be produced by proteolytic cleavage of the hinge in an intact antibody molecule. The light chains are constructed of two domains, variable (VL) and constant (CL), separated by a switch.

Disulfide bonds in the hinge region connect the two heavy chains. The light chains are coupled to the heavy chains by additional disulfide bonds.

The variable regions of both the heavy and light chains (VH) and (VL) lie at the "tips" of the Y, where they are positioned to react with antigen. This tip of the molecule is the side on which the N-termini of the amino acid sequences are located.

The stem of the Y projects in a way to efficiently mediate effector functions such as the activation of complement and interaction with Fc receptors, or ADCC and ADCP. Its CH2 and CH3 domains bulge to facilitate interaction with effector proteins. The C-terminus of the amino acid sequence is located on the opposite side of the tip, which can be termed "bottom" of the Y.

Two types of light chain, termed lambda (λ) and kappa (κ), are found in antibodies. A given immunoglobulin either has κ chains or λ chains, never one of each. No functional difference has been found between antibodies having λ or κ light chains.

Each domain in an antibody molecule has a similar structure of two beta sheets packed tightly against each other in a com-pressed antiparallel beta barrel. This conserved structure is termed the immunoglobulin fold. The immunoglobulin fold of constant domains contains a 3-stranded sheet packed against a 4-stranded sheet. The fold is stabilized by hydrogen bonding be-tween the beta strands of each sheet, by hydrophobic bonding between residues of opposite sheets in the interior, and by a disulfide bond between the sheets. The 3-stranded sheet comprises strands C, F, and G, and the 4-stranded sheet has strands A, B, E, and D. The letters A through G denote the sequential positions of the beta strands along the amino acid sequence of the immunoglobulin fold.

### Immunoglobulin variable domains (V domains)

The fold of variable domains has 9 beta strands arranged in two sheets of 4 and 5 strands. The 5-stranded sheet is structurally homologous to the 3-stranded sheet of constant domains, but contains the extra strands C' and C". The remainder of the strands (A, B, C, D, E, F, G) have the same topology and similar structure as their counterparts in constant domain immunoglobulin folds. A disulfide bond links strands B and F in opposite sheets, as in constant domains. The variable domains of both light and heavy immunoglobulin chains contain three hypervariable loops, or complementarity-determining regions (CDRs). The three CDRs of a V domain (CDR1, CDR2, CDR3) cluster at one end of the beta barrel. The CDRs are loops that connect beta strands B-C, C'-C", and F-G of the immunoglobulin fold. The residues in the CDRs vary from one immunoglobulin molecule to the next, imparting antigen specificity to each antibody.

The VL and VH domains at the tips of antibody molecules are closely packed such that the 6 CDRs (3 on each domain) cooperate in constructing a surface (or cavity) for antigen-specific binding. The natural antigen binding site of an antibody thus is composed of the loops which connect strands B-C, C'-C", and F-G of the light chain variable domain and strands B-C, C'-C", and F-G of the heavy chain variable domain.

### Scaffolds for protein engineering

Using the 3D structure of a protein as an aid for design, amino acid residues located on the surface of many proteins have been randomized using the core structure of the protein as scaffold. Examples for this strategy are reviewed in the following references and incorporated herein by reference: Binz et al. Nat Biotechnol. (2005) 23:1257-68; Hosse et al. Protein Sci. (2006) 15:14-27.

The basic principle of this technique is based on the observation that many proteins have a stable core, formed by specific arrangements of secondary structure elements such as beta sheets or alpha helices, which are interconnected by structures such as loops, turns, or random coils. Typically, these latter three structure elements are less crucial for the overall structure of the protein, and amino acid residues in these structure elements can be exchanged often without destroying the general fold of the protein. Naturally occurring examples for this design principle are the CDRs of immunoglobulin-like domains as can be found in antibodies, T-cell receptors and other molecules of the immunoglobulin superfamily. Artificial examples include lipocalins, ankyrins, kunitz domain inhibitor, knottin and other protein scaffolds.

T-cell receptors (TCRs) are important molecules of the immune system. Its extracellular domains are homologous with and structurally similar to an antibody Fab fragment.

T-cell receptors are expressed in nature on the surface of T-cells usually as alpha/beta and gamma/delta heterodimeric integral membrane proteins, each subunit comprising a short intracellular segment, a single transmembrane alpha-helix and two globular extracellular Ig-superfamily domains. The TCR-heterodimer is stabilized by an extracellular, membrane proximal, inter-chain disulphide bond (Immunobiology. 5th ed. Janeway, Charles A.; Travers, Paul; Walport, Mark; Shlomchik, Mark. New York and London: Garland Publishing; 2001). TCRs therefore have four extracellular domains, the two membrane proximal (C-terminal) domains, two of, which are constant, and the two N-terminal, two domains of which are variable. The variable domains are encoded by variable gene segments which are rearranged with junctional and constant gene segments(and diversity gene segments in the case of β-chains) to produce the TCR diversity observed in the mature immune system.

Both variable domains as well as constant domains of T-cell receptors are structurally similar to antibody domains and exhibit the typical "immunoglobulin fold":
Each domain has a similar structure of two beta sheets packed tightly against each other in a compressed antiparallel beta barrel. The immunoglobulin fold of TCR-constant domains (C-domains) contains a 3-stranded sheet packed against a 4-stranded sheet. The fold is stabilized by hydrogen bonding between the beta-strands of each sheet, by hydrophobic bonding between residues of opposite sheets in the interior, and by a disulfide bond between the sheets. The 3-stranded sheet strands are denoted A, F, and G , and the 4-stranded sheet comprises the strands B, C, D, and E. The letters A through G denote the sequential positions of the beta strands along the amino acid sequence of the immunoglobulin fold.

The fold of variable domains (V-domains) has 9 beta strands arranged in two sheets of 4 and 5 strands. The 5-stranded sheet is structurally homologous to the 3-stranded sheet of constant domains, but contains the extra strands C' and C". The remainder of the strands (A, B, C, D, E, F, G) have the same topology and similar structure as their counterparts in constant domain immunoglobulin folds. A disulfide bond links strands B and F in opposite sheets, as in constant domains.

The variable domains of both TCR chains contain three hypervariable loops, or complementarity-determining regions (CDRs). The three CDRs of a V-domain (CDR1, CDR2, CDR3) cluster at one end of the beta barrel. The CDRs are loops that connect beta strands B-C, C'-C", and F-G of the immunoglobulin fold. The residues in the CDRs vary from one TCR molecule to the next, imparting antigen specificity to each TCR.

The V-domains at the tips of TCR-molecules are closely packed such that the 6 CDRs (3 on each variable domain) cooperate in constructing a surface (or cavity) for antigen-specific binding. The natural antigen binding site of a TCR thus is composed of the loops which connect strands B-C, C'-C", and F-G of the light chain variable domain and strands B-C, C'-C", and F-G of the heavy chain variable domain. The extent of the specific contribution to antigen binding by the six CDR loops can vary between different TCRs.

It has been demonstrated that T-cell receptors have therapeutic and diagnostic potential and can be manipulated similarly to antibody molecules (e.g. Molloy et al. Curr Opin Pharmacol. (2005) 5:438-443; Boulter & Jakobsen (2005) Clin Exp Immunol. 142:454-460).

### Manipulation of the CDR-loops of immunoglobulin variable domains

A multitude of prior art documents show that the immunoglobulin like scaffold has been employed for the purpose of manipulating the existing antigen- or ligand-binding site, thereby introducing novel binding properties. More precisely, mainly the CDR regions have been engineered for antigen binding, in other words, in the case of the immunoglobulin fold, the natural antigen binding site has been modified in order to change its binding affinity or specificity. A vast body of literature exist which describes different formats of such manipulated immunoglobulins, frequently expressed in the form of complete antibodies, fusion products and/ or fragments such as single-chain Fv fragments (scFv), diabodies, minibodies, single domains or Fab fragments and the like, either displayed on the surface of phage particles or other viruses and cells or solubly expressed in various prokaryotic or eukaryotic expression systems. The techniques are reviewed e.g. in Holliger & Hudson, Nat. Biotechnol. (2005) 23:1126-36 and Hoogenboom, Nat. Biotechnol. (2005)23:1105-16.

### Framework or non-CDR-regions of immunoglobulin variable domains

The CDR-loops of an immunoglobulin variable domain define the antigen specificity. The rest of the molecule is termed framework (FR). These framework regions however are composed of beta-strand and loop structures, also called structural loops.

The loops which are not CDR-loops in a native immunoglobulin variable domain do not have antigen-binding or epitope-binding specificity but contribute to the correct overall folding of the immunoglobulin domain and consequently also to the correct positioning of the CDRs and to interaction between domains. These loops are named structural loops for the purpose of this invention.

Antibody variable domains in general have been manipulated for very many different reasons, such as the construction of various antibody formats, CDR grafting (i.e. grafting of specificity of a particular antibody into another framework; e.g. Jones et al. Nature (1986) 321: 522-525; Kashmiri et al. Methods (2005) 36:25-34), changing the surface of variable domains in order to make it more soluble and stable (e.g. Ewert et al. Methods (2004) 34:184-99), to render it monomeric (e.g. Dottorini et al. Biochemistry (2004) 43:622-628))or to study the interaction between variable domains (e.g. Masuda et al. FEBS J. (2006) 273:2184-94). Many of those manipulations have involved changes in the framework region of the molecule; some amino acid mutations within structural loops of the variable domain have been performed.

The influence of remote framework regions on CDR-loop positioning is evident from CDR grafting results which show that mutation of framework amino acids frequently is necessary to regain antigen binding after grafting of CDRs from one framework to another (e.g. Wu et al. J. Mol. Biol. (1999) 294:151-162).

Simon & Rajwesky (Protein Sci. (1992) 5:229-234) have studied the effects of a four residue insertion into the FR3 loop of the heavy chain variable region from the anti-NP antibody B1-8. The insertion mutant was obtained as secreted antibody without major defects in biosynthesis, indicating that antibody variable domains can accommodate length variation not only in complementarity determining regions (CDRs), but also in framework region (FR) loops. In this case the original antigen binding site formed by the CDR-loops was not affected by the modification in a neighbouring structural loop.

### Molecules with grafted binders of FcRn

WO9743316 describes the extension of the half life of biologically active molecules through structural modification such that they include a binding molecule to the IgG protection receptor FcRp, which is identical to FcRn. For example, the structure of the physiologically active molecules can be modified to include a portion of an antibody which is raised against FcRp, i.e. a Fab fragment, an Fv fragment, a Fab' fragment or a F(ab')2 fragment. Alternatively, antibody regions that are known to be involved in FcRn receptor binding, such as the amino acid sequences of the CH2 and CH3 domain, known as the Brambell motif, can be engineered into physiologically active molecules to extend their half-life. Binding of those antibody fragments or motifs is preferably pH dependent, thus binding to the IgG protection receptor at acidic pH, e.g., a pH of about 6.5 or lower, and release from the IgG protection receptor at neutral pH, e.g., a pH of about 7.0 to about 7.5. The suitable antibody fragments or motifs are then fused or coupled to the physiologically active molecules to extend their half-lives.

The resulting physiologically active molecule, however, through the conjugation of antibody fragments, has a significantly changed structure, far away from the natural form that can be easily tolerated by the human immune system.

Likewise US5739277 discloses the grafting of Fc region sequences to polypeptides, so to engineer a salvage receptor binding epitope of an Fc region of an IgG into the polypeptide. The FcRn binding site is thus transferred from an Fc region to a polypeptide of interest. Again the resulting structure significantly differs from the natural form of an immunoglobulin.

Another way of grafting or conjugating FcRn binders is described by WO9734631, which discloses immunoglobulin-like domains with increased half-lives and the transport of serum proteins and antibodies mediated by the Fc receptor, FcRn. Functional agents include or are bound to a molecule or a moiety that binds to FcRn in a pH dependent manner, such that binding affinity is strong at about pH 6 to about pH 6.5 relative to binding at pH 7.4. Thereby the agent has an increased half-live. Stabilization tags for such agents are provided that bind to FcRn in a pH dependent manner.

US6030613 refers to the receptor specific transepithelial transport of therapeutics. Antigens are coupled to molecules that bind to the FcRn receptor for delivery across epithelial barriers. Exemplary molecules are Fc fragments that bind to Fc receptors.

WO02060919 A2 refers to a method of improving the affinity of the FcRn binding region of IgG constant domains through modifications, and molecules with extended half-lives, by virtue of the presence of such an IgG constant domain or FcRn-binding portion thereof. In particular, the disclosure relates to either modified IgGs including the modified constant domain with increased affinity to FcRn, or to non-immunoglobulin proteins or non-protein agents that are fused or conjugated to, or engineered to contain an IgG constant domain having an increased affinity to FcRn.

Phage display has been used to select for a number of Fc mutants with better binding to FcRn at pH 6.0 (Dall'Acqua et al.The Journal of Immunology, 2002, 169:5171-5180). Recently, Hinton and coworkers developed human Fc mutants with increased FcRn binding affinity that showed an increased half-life in primates (Hinton et al., The Journal of Immunology, 2006, 176: 346-356), extending similar results achieved in mice by Ward and coworkers (Ghetie et al., Nat Biotechnol., 1997, 15:637-40).

To reduce the chances of interfering with the pH dependency of Fc binding, a homology model of the human Fc/FcRn complex was visually inspected to guide the selection of individual residues distant from those known to be involved in pH dependent binding.

WO0009560 A2 discloses the generation of modified molecules, in particular antibody molecules, with increased serum half-life. The antibody molecules that contain an FcRn binding region are physically linked to a second FcRn binding domain. FcRn binding moieties are used for this purpose, whether generated directly from Fc of IgG, derived from Fc of IgG and screened, or simply identified through screening.

The modified antibody molecules or biologically active agents of the prior art are commonly produced to include an extra FcRn binding site grafted or conjugated to a molecule so to improve its half-live and transepithelial transport. However, the resulting molecules are significantly denaturated, i.e. lacking their natural structure. With regard to immunoglobulins it is however critical to provide the molecule in a native structure because they should be easily tolerated by the human immune system. Immunoglobulins or immunoglobulin-like molecules that are used for immunotherapies, usually are administered in large amounts and/or frequently administered, thus afflicted with a high risk of immunogenic side reactions.

### Binding to FcRn through structural loops

Recent work described in WO 06072620 refers to a method for engineering an immunoglobulin comprising modifications in a structural loop region and determining the binding of said immunoglobulin to an antigen. This method employs randomization techniques to modify structural loops and to create new binding sites for antigens. The disclosure further refers to the method of increasing half-life of immunoglobulins, by randomization of structural loops to create novel binding sites. Libraries of mutant e.g. CH1-, CH2-, CH3-, CH4-, Cκ- or Cλ-domains may be screened for FcRn binding.

In order to modulate the properties of a molecule consisting of or containing a variable domain, structural loops have been mutated to bind serum proteins or complement proteins according to A1145/2006. Binders to FcRn or serum albumin may be selected from libraries of mutant variable domains. Either the isolated structural loops or molecules containing the structural loops in the context of an immunoglobulin are used to design molecules with increased half life in vivo.

Though the randomization of structural loops has been proven to engineer new antigen binding sites, any transcytotic properties of the selected binders have not been described.

It is the object of the present invention to engineer new transcytotic immunoglobulins with a native structure.

### Summary of the invention

The object is achieved through the immunoglobulin and modular antibody according to the present invention and its specific embodiments.

According to the invention there is provided a transcytotic modular antibody containing a de novo pH dependent binding site for a transport protein. Thereby it has proven that the modular antibody can pass an endothelial cell layer. Preferably the modular antibody exhibits a prolonged half-life.

The modular antibody according to the invention preferably is provided with a binding site having a specificity for a transport protein that mediates endocytotic and/or transcytotic trafficking. The preferred transport protein is selected from the group consisting of FcRn, Albumin and IgG. Binding of these molecules in a pH dependent manner has proven to prolong the in vivo half-life of an antibody.

According to the invention the modular antibody may further contain a natural binding site for binding a transport protein or other binding sites that are inherently contained in an IgG molecule, besides the novel pH dependent binding site. It is particular preferred that the modular binding site further contains at least one binding site against one of FcRn and Protein A.

The pH dependency of the binding site of the modular antibody of the invention is preferably characterized by the binding of the transport protein at pH 6-7 with higher affinity than at pH 7.1-7.6.

The modular antibody according to the invention is provided preferably as an immunoglobulin, a member of the immunoglobulin superfamily, or proteins with immunoglobulin-like structure, or parts thereof. For instance, a modular antibody according to the invention may be a member of the immunoglobulin superfamily selected from the group consisting of intact IgG, Fab, Fc, scFv and dAb. Other specific embodiments relate to proteins with immunoglobulin-like modular structure, which are preferably selected from the group consisting of fibronectin, TCR, transferrin, CTLA-4, single-chain antigen receptors, e.g. those related to T-cell receptors and antibodies, antibody mimetics, adnectins, molecules based on anticalins, phylomers, avimers, affibodies, ankyrin repeats, Kunitz domains, PDZ-domains, scorpio toxins immunity proteins, CTLA-4, Knottins, Versabodies, Green Fluorescent Protein and other non-antibody protein scaffolds with antigen binding properties.

Though the modular antibody according to the invention is engineered to contain a pH dependent binding site, it still has a native structure. In a specific embodiment it may be provided as a composite molecule, preferably it is composed of at least one protein domain in the native form, preferably an antibody domain.

The pH dependent binding site for the transport protein is preferably located in the framework region. Most preferred sites are presented by at least a structural loop of a protein domain, preferably a constant or variable antibody domain.

The modular antibody according to the invention may be provided as a native molecule, or else conjugated to biologically active structures, or fused therewith.

According to the invention it turned out that specific amino acids are preferably introduced into the modular antibody, by substitution and/or insertion techniques. Specific embodiments refer to binding site including at least one of acidic or basic amino acids, preferably at least two of them, within sterical or spatial proximity.

Exemplary methods of producing the modular antibody according to the invention refer to modifying parent molecules by specific engineering techniques. A method according to the invention may produce a modular antibody according to the invention by engineering a molecule containing an antibody domain to modify a structural loop and determining the binding of said molecule to an epitope of a transport protein at a first pH, wherein the unmodified molecule does not significantly bind to said transport protein at a second pH, which comprises the steps of:
- providing a nucleic acid encoding a molecule containing an antibody domain comprising at least one structural loop region,
- modifying at least one nucleotide residue of at least one of said structural loop regions,
   - transferring said modified nucleic acid in an expression system,
   - expressing said modified molecule,
- contacting the expressed modified molecule with an epitope of a transport protein, and
- determining whether said modified molecule binds to said epitope in a pH dependent manner.

It is however understood that the modular antibodies according to the invention can be reengineered or produced based on a known amino acid sequence, by any appropriate technique of recombinant production or synthesis methods.

The method according to the invention preferably employs modular antibody technology. This enables the construction of antibodies built from antibody domains in a modular way. The preferred engineering methods relate to engineering of a molecule containing an antibody domain to modify a structural loop and determining the binding of said molecule to an epitope of a transport protein at a first pH, wherein the unmodified molecule does not significantly bind to said transport protein at a second pH, comprising the steps of:
- providing a nucleic acid encoding a molecule containing an antibody domain comprising at least one structural loop region,
- modifying at least one nucleotide residue of at least one of said structural loop regions,
- transferring said modified nucleic acid in an expression system,
- expressing said modified molecule,
- contacting the expressed modified molecule with an epitope of a transport protein, and
- determining whether said modified molecule binds to said epitope in a pH dependent manner.

The preferred method employs the modification of the nucleic acid by one of randomization, nucleotide sequence grafting or fusion techniques. However, other methods that relate to grafting of peptides or amino acid sequences onto the modular antibody, specifically within the structural loop region, may be preferred as well. One of the preferred embodiments thus refers to a method of conjugating a peptide to a molecule containing an antibody domain, wherein the peptide is binding to a transport protein at a first pH, wherein the unmodified molecule does not significantly bind to said transport protein at a second pH. Suitable peptides can be selected from respective peptide libraries.

The modular antibody according to the invention are preferable used to design and prepare dedicated protein libraries, whereby the modular antibody containing the pH dependent binding site forms the basis for a library to select members with additional functionality or pharmacologic properties.

Libraries according to the invention comprise at least 10 modular antibodies according to the invention, preferably at least 100, more preferred at least 1000, more preferred at least 10⁴, more preferred at least 10⁵, more preferred at least 10⁶, more preferred at least 10⁷, more preferred at least 10⁸, more preferred at least 10⁹, more preferred at least 10¹⁰, more preferred at least 10¹¹, up to 10¹².

The libraries according to the invention may specifically contain with mutations to obtain at least one acidic or basic amino acid in a structural loop region, preferably at least two, more preferred at least three, wherein the basic amino acids are most preferred.

Specific libraries according to the invention contain modular antibodies with at least two proximal acidic or basic amino acids. Proximal amino acids are located within sequence proximity of less than 12 amino acids, more preferred less than 6 amino acids. Proximity can however also be achieved through steric or spatial proximity of the beta sheets and loops that are located in near distance of less than 50 Angström, preferably less than36 Angström, more preferably less than 25 Angström, most preferably less than 20 Angström.

Particular embodiments refer to libraries that contain modular antibodies with at least two structural loops with at least one acidic or basic amino acids in the at least two structural loops, preferably at least two, more preferred at least three, wherein the basic amino acids are most preferred.

A library according to the invention may be designed as a dedicated library that contains at least 50% specific formats, preferably at least 60%, more preferred at least 70%, more preferred at least 80%, more preferred at least 90%, or those that mainly consist of specific antibody formats. Specific antibody formats are preferred, such that the preferred library according to the invention it is selected from the group consisting of a VH library, VHH library, Vkappa library, Vlambda library, Fab library, a CH1/CL library and a CH3 library. Libraries characterized by the content of composite molecules containing more than one antibody domains, such as an IgG library or Fc library are specially preferred. Other preferred libraries are those containing T-cell receptors, forming T-cell receptor libraries.

Further embodiments relate to libraries of immunoglobulin-like molecules or other modular protein scaffolds, for instance, libraries selected from the group consisting of T-cell receptor library, fibronectin library, avimer library, anticalin library, ankyrin repeat library, Kunitz domain libraries, scorpio toxin based libraries and Versabody^{™} based libraries.

In some cases it is libraries are preferred that contain a significant portion of library members with specific mutations. Those specifically designed libraries preferably contain members with basic amino acids in structural loops, preferably the C-terminal loops, which portion or share is higher than 10%, preferably at least 20%, more preferred at least 30%, more preferred at least 40%, more preferred at least 50%, more preferred at least 60%, more preferred at least 70%, more preferred at least 80%, more preferred at least 90%, more preferred at least 95%, up to 99%.

The preferred Fc library according to the invention further contains a significant portion of stable library members with specific mutations. Those specifically designed libraries preferably contain members with binding properties to Protein A, which portion is higher than 10%, preferably at least 20%, more preferred at least 30%, more preferred at least 40%, more preferred at least 50%, more preferred at least 60%, more preferred at least 70%, more preferred at least 80%, more preferred at least 90%, more preferred at least 95%, up to 99%.

According to the invention there is further provided a method for specifically isolating a transcytotic modular antibody binding to a transport protein from a library containing modular antibodies according to the invention, which comprises the steps of:
a. contacting said library with a test sample containing said transport protein at an acidic pH, isolating the specific antibody/transport protein complex formed by the binders to said transport protein from the non-binders, and
b. treating said specific antibody/transport protein complex at a neutral to basic pH to release the pH dependent binders.

The test sample may contain the transport protein ether in soluble form or on a solid phase, including transport proteins bound to carriers or cell-bound transport proteins, like membrane proteins in the natural context.

In this regard there is further provided a kit of binding partners containing
a. a library containing modular antibodies according to the invention, and
b. a transport protein.

Thereby the transport protein of the kit according to the invention can be used specifically for selecting a modular antibody from a library, employing standard panning techniques.

It can be shown that by engineering a de novo pH dependent binding site for a transport protein into a modular antibody the serum half-life or biological half-life of such a modular antibody can be prolonged, as determined by in vitro, ex vitro or in vivo assays.

The modular antibody with prolonged half-life may be used as such for diagnostic or therapeutic purposes, but may also serve as a building block to prolong the half-life of a biologically active molecule, by conjugating with or fusing to the modular antibody according to the invention. The biologically active molecule is any drug that is advantageously kept in the patients' circulation or at local sites for a prolonged period of time. Those molecules are typically drugs with physiological function or therapeutics based on biologicals, including peptides, polypeptides or proteins, like other modular antibodies or immunoglobulins, or else small molecules. Standard recombinant or fusion techniques, or conjugation methods can be employed to achieve binding of the modular antibody building block to the biologically active molecule. As a fusion or conjugation product a composite molecule is obtained that may use the modular antibody as carrier for adding the prolonged half-life property to the biologically active molecule, which carrier function is related to targeting antigens or just as a building block to improve the molecules pharmacologic properties, functionality or specificity.

Pharmaceutical preparations containing a transcytotic modular antibody according to the invention may be used for the immunotherapy of a patient. Therapeutic methods according to the invention typically refer to passive or active immunotherapies using appropriate formulation and administration schedules.

Specifically the transcytotic modular antibody according to the invention is formulated for systemic or local, parenteral, such as i.v. or s.c. administration, or else topical or mucosal administration, e.g. by sublingual or oral application.

### More detailed description of the invention

According to the invention there is provided a transcytotic modular antibody containing a de novo pH-dependent binding site for a transport protein. A novel pH-dependent binding site is thus created according to the invention within the antibody structure in a modular way. This way of engineering is preferred to keep the native antibody structure, preferably without loosing advantageous antibody properties, like antigen binding, Fc receptor binding and/or Protein A binding. It is surprising that the modification of only a few amino acids within the native structure of an antibody can bring about the pH dependency of binding. In particular when structural loops of immunoglobulins or immunoglobulin-like molecules are mutated, it surprisingly turns out that only a few structure sparing mutations are necessary to create the novel or extra pH dependent binding site for a transport protein, like FcRn, serum albumin or immune serum globulins. By means of minimal conservative mutations, the novel binding site can be created while preserving the overall structure of the modular antibody.

pH-dependency has proven to be essential for the endocytotic or transcytotic property of a modular antibody. This can be shown in a respective in vitro or ex vivo model using cell layers of endothelial or epithelial cells. Transcytosis, which includes endocytosis, is key to the beneficial pharmacodynamic and pharmacokinetic properties, including better tissue penetration and half-life of a molecule. With respect to antibodies and modular antibodies that are based on antibody domains engineered in a modular way, the pharmacokinetic properties can be improved by virtue of a novel pH dependent binding site with specificity for a transport protein according to the invention. A de novo binding site is thus engineered into the antibody according to the invention in a modular way, so to avoid or exclude the destruction of the native structure of the protein. By utilizing antibody building blocks or modules with modifications at various different sites of a protein, libraries are preferably created by a "domain shuffling" technique, which can be selected for pH dependent binding of the transport protein. Members of the libraries with the desired binding properties are then characterized for the native structure of the molecule to be maintained. Selected members of such a library contain the modifications within the modular antibody, which modifications have built the new binding site, besides any existing binding sites, for binding a transport protein in a pH dependent manner.

"Modular antibodies" are defined as antigen-binding molecules, like human antibodies, composed of at least one polypeptide module or protein domain in the natural form. It is believed that one of the most important characteristics of the modular antibody is the natural structure, which is maintained even after modifying the molecule so to engineer new binding sites into the molecule. Thus, preferable properties of modular antibodies, such as Protein A binding, or binding of one or more antigens are still retained. The conservative modification also allows for the presentation of the antigen-binding molecule in a non-immunogenic form, which enables the frequent administration to test animals or patients without an undesired immune reaction.

The term "modular antibodies" includes antigen-binding molecules that are either immunoglobulins, immunoglobulin-like proteins, or other proteins exhibiting modular formats and antigen-binding properties similar to immunoglobulins or antibodies, which can be used as antigen-binding scaffolds, preferably based on human proteins.

The invention preferably refers to complete antibody molecules or part of antibodies, such as IgG, IgA, IgM, IgD, IgE and the like. The modular antibodies of the invention may also be a functional antibody fragment such as Fab, Fab₂, scFv, Fv, Fc, Fcab^{™}, or parts thereof, or other derivatives or combinations of the immunoglobulins such as minibodies, domains of the heavy and light chains of the variable region (such as dAb, Fd, VL, including Vlambda and Vkappa, VH, VHH) as well as mini-domains consisting of two beta-strands of an immunoglobulin domain connected by at least two structural loops, as isolated domains or in the context of naturally associated molecules. A particular embodiment of the present invention refers to the Fc fragment, Fcab^{™}, of an antibody molecule, either as antigen-binding Fc fragment through modifications of the amino acid sequence or as conjugates to receptors or other antigen-binding modules, such as scFv.

The term "immunoglobulin" as used according to the present invention is defined as polypeptides or proteins that may exhibit mono- or bi- or multi-specific, or mono-, bi- or multivalent binding properties, preferably at least two, more preferred at least three specific binding sites for epitopes of e.g. antigens, effector molecules or proteins either of pathogen origin or of human structure, like self-antigens including cell-associated or serum proteins. The term immunoglobulin according to the invention also includes functional fragments, such as Fc, Fab, scFv, single chain dimers of CH/CL domains, Fv, Fc, or other derivatives or combinations of the immunoglobulins. The definition further includes domains of the heavy and light chains of the variable region (such as dAb, Fd, VI, Vk, Vh) and the constant region or individual domains of an intact antibody such as CH1, CH2, CH3, CH4, Cl and Ck, as well as mini-domains consisting of at least two beta-strands of an immunoglobulin domain connected by a structural loop.

Antibody or immunoglobulin domains may be modified according to the present invention (as used herein the terms immunoglobulin and antibody are interchangeable) which modifications are preferably effected in immunoglobulin domains or parts thereof that contain a structural loop, or a structural loop of such domains. The immunoglobulins can be used as isolated peptides or as combination molecules with other peptides. In some cases it is preferable to use a defined modified structural loop or a structural loop region, or parts thereof, as isolated molecules for binding or combination purposes. The peptides are homologous to immunoglobulin variable domain sequences, and are preferably at least 5 amino acids long, more preferably at least 10 or even at least 50 or 100 amino acids long, and constitute at least partially the structural loop region or the non-CDR loop region of the immunoglobulin domain. Preferably the peptides exclude those insertions that are considered non-functional amino acids, hybrid or chimeric CDR-regions or CDR-like regions and/or canonical structures of CDR regions. The binding characteristics relate to predefined epitope binding, affinity and avidity.

The term "immunoglobulin-like molecule" as used according to the invention refers to any antigen-binding human protein, which has a domain structure that can be built in a modular way. Immunoglobulin-like molecules as preferably used for the present invention are T-cell receptors (TCR), fibronectin, transferrin, CTLA-4, single-chain antigen receptors, e.g. those related to T-cell receptors and antibodies, antibody mimetics, adnectins, anticalins, phylomers, repeat proteins such as ankyrin repeats, avimers, Versabodies^{™}, scorpio toxin based molecules, and other non-antibody protein scaffolds with antigen binding properties.

Ankyrin repeat (AR), armadillo repeat (ARM), leucine-rich repeat (LRR) and tetratricopeptide repeat (TPR) proteins are the most prominent members of the protein class of repeat proteins. Repeat proteins are composed of homologous structural units (repeats) that stack to form elongated domains. The binding interaction is usually mediated by several adjacent repeats, leading to large target interaction surfaces.

Avimers contain A-domains as strings of multiple domains in several cell-surface receptors. Domains of this family bind naturally over 100 different known targets, including small molecules, proteins and viruses. Truncation analysis has shown that a target is typically contacted by multiple A-domains with each domain binding independently to a unique epitope. The avidity generated by combining multiple binding domains is a powerful approach to increase affinity and specificity, which these receptors have exploited during evolution.

Anticalins are engineered human proteins derived from the lipocalin scaffold with prescribed binding properties typical for humanized antibodies. Lipocalins comprise 160-180 amino acids and form conical b-barrel proteins with a ligand-binding pocket surrounded by four loops. Small hydrophobic compounds are the natural ligands of lipocalins, and different lipocalin variants with new compound specificities (also termed 'anticalins') could be isolated after randomizing residues in this binding pocket.

Antigen receptors contain a single variable domain and are 20% smaller than camelid single domain antibodies.

Phylomers are peptides derived from biodiverse natural protein fragments.

The modular antibody according to the invention is possibly further combined with one or more modified immunoglobulins or with unmodified immunoglobulins, or parts thereof, to obtain a combination immunoglobulin. Combinations are preferably obtained by recombination techniques, but also by binding through adsorption, electrostatic interactions or the like, or else through chemical binding with or without a linker. The preferred linker sequence is either a natural linker sequence or functionally suitable artificial sequence.

It is understood that the term "modular antibody", "immunoglobulin", "immunoglobulin-like proteins" includes a derivative thereof as well. A derivative is any combination of one or more modular antibodies of the invention and or a fusion protein in which any domain or minidomain of the modular antibody of the invention may be fused at any position of one or more other proteins (such as other modular antibodies, immunoglobulins, ligands, scaffold proteins, enzymes, toxins and the like). A derivative of the modular antibody of the invention may also be obtained by association or binding to other substances by various chemical techniques such as covalent coupling, electrostatic interaction, di-sulphide bonding etc. The other substances bound to the immunoglobulins may be lipids, carbohydrates, nucleic acids, organic and anorganic molecules or any combination therof (e.g. PEG, prodrugs or drugs). A derivative is also an antibody with the same amino acid sequence but made completely or partly from non-natural or chemically modified amino acids.

The engineered molecules according to the present invention will be useful as stand-alone proteins as well as fusion proteins or derivatives, most typically fused in such a way as to be part of larger antibody structures or complete antibody molecules, or parts thereof such as Fab fragments, Fc fragments, Fv fragments and others. It will be possible to use the engineered proteins to produce molecules which are monospecific, bispecific, trispecific, and maybe even carry more specificities at the same time, and it will be possible at the same time to control and preselect the valency of binding at the same time according to the requirements of the planned use of such molecules.

The term "native structure" refers to molecules that retain the main structural features upon modification. Thus, even after mutating the amino acid sequence (primary structure) of polypeptides or proteins, its secondary or tertiary structure is alike the unmodified parent molecule, so that important characteristics, like binding to targets, epitopes, antigens or natural ligands, among them T-cell receptors, Fc-receptors, or other ligands like Protein A, is maintained. Preferably binding of the parent molecule to an epitope is retained in a way that the binding affinity is not significantly decreased, meaning that the dissociation constant Kd is not significantly increased, so to preferably provide a high affinity binder with a Kd in the range of 10⁻⁹ to 10⁻¹². More preferably the Kd difference to the parent molecule is less than 10³, most preferably less thank 10², or even less than 10¹. It may however sometimes be desirable to increase the Kd through the modification, so to obtain a binder with moderate binding affinity of 10⁻⁷ to 10⁻⁸. Thus a Kd difference due to an increased binding affinity of at least 10¹, preferably at least 10², most preferably at least 10³ would be considered a preferred embodiment of the invention.

According to the present invention, the modular antibody exerts one or more binding regions to antigens, including transport proteins. Antigen binding sites to one or more antigens may be introduced into a structural loop region of a given antibody domain, either of a variable or constant domain structure. The antigens may be naturally occurring molecules or chemically synthesized molecules or recombinant molecules, either in solution or in suspension, e.g. located on or in particles such as solid phases, on or in cells or on viral surfaces. It is preferred that the binding of an immunoglobulin to an antigen is determined when the antigen is still adhered or bound to molecules and structures in the natural context. Thereby it is possible to identify and obtain those modified immunoglobulins that are best suitable for the purpose of diagnostic or therapeutic use.

The term "antigen" as used herein shall comprise molecules selected from the group consisting of allergens, tumor associated antigens, self antigens including cell surface receptors, enzymes, Fc-receptors, FcRn, HSA, IgG, interleukins or cytokines, proteins of the complement system, transport proteins, serum molecules, bacterial antigens, fungal antigens, protozoan antigen and viral antigens, also molecules responsible for transmissible spongiform encephalitis (TSE), such as prions, infective or not, and markers or molecules that relate to inflammatory conditions, such as pro-inflammatory factors, multiple sclerosis or alzheimer disease, or else haptens. Antigens can be targeted and bound by the modular antibody through at least part of a structural loop or CDR loop.

The modular antibodies of the invention may specifically bind to any kind of ligands or binding molecules, in particular to proteinaceous molecules, proteins, peptides, polypeptides, nucleic acids, glycans, carbohydrates, lipids, small and large organic molecules, inorganic molecules. Typically, the modular antibodies may comprise loop regions whereby some of the loop regions may specifically bind to different molecules or epitopes.

According to a preferred embodiment of the present invention the antigen or the molecule binding to the modified structural loop region is selected from the group consisting of tumor associated antigens, in particular EpCAM, tumor-associated glycoprotein-72 (TAG-72), tumor-associated antigen CA 125, Prostate specific membrane antigen (PSMA), High molecular weight melanoma-associated antigen (HMW-MAA), tumor-associated antigen expressing Lewis Y related carbohydrate, Carcinoembryonic antigen (CEA), CEACAM5, HMFG PEM, mucin MUC 1, MUC 18 and cytokeratin tumor-associated antigen, bacterial antigens, viral antigens, allergens, allergy related molecules IgE, cKIT and Fc-epsilon-receptorI, IRp60, IL-5 receptor, CCR3, red blood cell receptor (CR1), human serum albumin, mouse serum albumin, rat serum albumin, neonatal Fc-gamma-receptor FcRn, Fc-gamma-receptors Fc-gamma RI, Fc-gamma-RII, Fc-gamma RIII, Fc-alpha-receptors, Fc-epsilon-receptors, fluorescein, lysozyme, toll-like receptor 9, erythropoietin, CD2, CD3, CD3E, CD4, CD11, CD11a, CD 14, CD16, CD18, CD19, CD20, CD22, CD23, CD25, CD28, CD29, CD30, CD32, CD33 (p67 protein), CD38, CD40, CD40L, CD52, CD54, CD56, CD64, CD80, CD147, GD3, IL-1, IL-1R, IL-2, IL-2R, IL-4, IL-5, IL-6, IL-6R, IL-8, IL-12, IL-15, IL-17, IL-18, IL-23, LIF, OSM, interferon alpha, interferon beta, interferon gamma; TNF-alpha, TNFbeta2, TNFalpha, TNFalphabeta, TNF-R1, TNF-RII, FasL, CD27L, CD30L, 4-1BBL, TRAIL, RANKL, TWEAK, APRIL, BAFF, LIGHT, VEG1, OX40L, TRAIL Receptor-1, A1 Adenosine Receptor, Lymphotoxin Beta Receptor, TACI, BAFF-R, EPO; LFA-3, ICAM-1, ICAM-3, integrin beta1, integrin beta2, integrin alpha4/beta7, integrin alpha2, integrin alpha3, integrin alpha4, integrin alpha5, integrin alpha6, integrin alphav, alphaVbeta3 integrin, FGFR-3, Keratinocyte Growth Factor, GM-CSF, M-CSF, RANKL, VLA-1, VLA-4, L-selectin, anti-Id, E-selectin, HLA, HLA-DR, CTLA-4, T cell receptor, B7-1, B7-2, VNRintegrin, TGFbetal, TGFbeta2, eotaxin1, BLyS (B-lymphocyte Stimulator), complement C5, IgE, IgA, IgD, IgM, IgG, factor VII, CBL, NCA 90, EGFR (ErbB-1), Her2/neu (ErbB-2), Her3 (ErbB-3), Her4 (ErbB4), Tissue Factor, VEGF, VEGFR, endothelin receptor, VLA-4, carbohydrates such as blood group antigens and related carbohydrates, Galili-Glycosylation, Gastrin, Gastrin receptors, tumor associated carbohydrates, Hapten NP-cap or NIP-cap, T cell receptor alpha/beta, E-selectin, P-glycoprotein, MRP3, MRP5, glutathione-S-transferase pi (multi drug resistance proteins), alpha-granule membrane protein(GMP) 140, digoxin, placental alkaline phosphatase (PLAP) and testicular PLAP-like alkaline phosphatase, transferrin receptor, Heparanase I, human cardiac myosin, Glycoprotein IIb/IIIa (GPIIb/IIIa), human cytomegalovirus (HCMV) gH envelope glycoprotein, HIV gp120, HCMV, respiratory syncital virus RSV F, RSVF Fgp, VNRintegrin, Hep B gp120, CMV, gpIIbIIIa, HIV IIIB gp120 V3 loop, respiratory syncytial virus (RSV) Fgp, Herpes simplex virus (HSV) gD glycoprotein, HSV gB glycoprotein, HCMV gB envelope glycoprotein, Clostridium perfringens toxin and fragments thereof.

Preferably, the antigen is selected from the group consisting of pathogen antigen, tumor associated antigen, enzyme, substrate, self antigen, organic molecule or allergen. More preferred antigens are selected from the group consisting of viral antigens, bacterial antigens or antigens from pathogens of eukaryots or phages. Preferred viral antigens include HAV-, HBV-, HCV-, HIV I-, HIV II-, Parvovirus-, Influenza-, HSV-, Hepatitis Viruses, Flaviviruses, Westnile Virus, Ebola Virus, Pox-Virus, Smallpox Virus, Measles Virus, Herpes Virus, Adenovirus, Papilloma Virus, Polyoma Virus, Parvovirus, Rhinovirus, Coxsackie virus, Polio Virus, Echovirus, Japanese Encephalitis virus, Dengue Virus, Tick Borne Encephalitis Virus, Yellow Fever Virus, Coronavirus, respiratory syncytial virus, parainfluenza virus, La Crosse Virus,Lassa Virus,Rabies Viruse, Rotavirus antigens; preferred bacterial antigens include Pseudomonas-, Mycobacterium-, Staphylococcus-, Salmonella-, Meningococcal-, Borellia-, Listeria, Neisseria-, Clostridium-, Escherichia-, Legionella-, Bacillus-, Lactobacillus-, Streptococcus-, Enterococcus-, Corynebacterium-, Nocardia-, Rhodococcus-, Moraxella-, Brucella, Campylobacter-, Cardiobacterium-, Francisella-, Helicobacter-, Haemophilus-, Klebsiella-, Shigella-, Yersinia-, Vibrio-, Chlamydia-, Leptospira-, Rickettsia-, Mycobacterium-, Treponema-, Bartonella- antigens. Preferred eukaryotic antigens of pathogenic eukaryotes include antigens from Giardia, Toxoplasma, Cyclospora, Cryptosporidium, Trichinella, Yeasts, Candida, Aspergillus, Cryptococcus,Blastomyces, Histoplasma, Coccidioides.

In a preferred embodiment the modular antibody is binding with its modified structural loops specifically to at least two such epitopes that are identical or differ from each other, either of the same antigen or of different antigens, one of the antigens being a transport protein.

For example, the method according to the invention refers to engineering a modular antibody that is binding specifically to at least one first epitope and comprising modifications in each of at least two structural loop regions, and determining the specific binding of said at least two loop regions to at least one second epitope, the second epitope being derived from a transport protein, wherein the unmodified structural loop region (non-CDR region) does not specifically bind to said at least one second epitope. Thus, an antibody or antigen-binding structure specific for a first antigen may be improved by adding another valency or specificity against a second antigen, which specificity may be identical targeting different epitopes or the same epitope, to increase valency or to obtain bi-, oligo- or multispecific molecules.

The term "transport protein" or "membrane transport protein", or simply "transporter", as used herein, is a protein involved in the movement of macromolecules, such as a modular antibody across a biological membrane. Transport proteins are integral membrane proteins; that is they exist within and span the membrane across which they transport substances. The proteins may assist in the movement of substances by facilitated diffusion or active transport. In this regard proteins are also considered as transport proteins that mediate the passage through biological membranes, such as serum proteins, like albumin or immunoglobulins or IgG, which bind to receptors of endothelial cells, e.g. FcRn, with transcytotic function. pH dependent transcytotis may result in a one-way movement of macromolecules, i.e. enabling transcytotic trafficking, but also in both directions. This two-way movement, resulting in possible endocytotic trafficking besides the transcytotic effect, enables the possible transient internalization of the macromolecule within cellular membranes, with the effect of protection against proteolytic degradation and potential half life prolongation. It is understood that transcytotis in most cases includes endocytosis.
A vesicular transport protein is a membrane protein, which uses vesicles to move the contents of the cell.
The term antigen as used according to the present invention shall in particular include all antigens and target molecules capable of being recognised by a binding site of a modular antibody. The antigen is either recognized as a whole target molecule or as a fragment of such molecule, especially substructures of targets, generally referred to as "epitopes".

Specifically preferred antigens as targeted by the modular antibody according to the invention are those antigens or molecules, which have already been proven to be or are capable of being immunogenic or antigenic, bound by immune response factors, or else immunologically or therapeutically relevant, especially those, for which a clinical efficacy has been tested.

The term "epitope" as used herein according to the present invention shall mean a molecular structure which may completely make up a specific binding partner or be part of a specific binding partner to a binding site of modular antibody or an immunoglobulin of the present invention. The term epitope may also refer to haptens.

Chemically, an epitope may either be composed of a carbohydrate, a peptide, a fatty acid, an organic, biochemical or inorganic substance or derivatives thereof and any combinations thereof. If an epitope is a polypeptide, it will usually include at least 3 amino acids, preferably 8 to 50 amino acids, and more preferably between about 10-20 amino acids in the peptide. There is no critical upper limit to the length of the peptide, which could comprise nearly the full length of a polypeptide sequence of a protein. Epitopes can be either linear or conformational epitopes. A linear epitope is comprised of a single segment of a primary sequence of a polypeptide chain. Linear epitopes can be contiguous or overlapping. Conformational epitopes are comprised of amino acids brought together by folding of the polypeptide to form a tertiary structure and the amino acids are not necessarily adjacent to one another in the linear sequence.

Specifically, epitopes are at least part of diagnostically relevant molecules, i.e. the absence or presence of an epitope in a sample is qualitatively or quantitatively correlated to either a disease or to the health status of a patient or to a process status in manufacturing or to environmental and food status. Epitopes may also be at least part of therapeutically relevant molecules, i.e. molecules which can be targeted by the specific binding domain which changes the course of the disease.

Preferred epitopes of transport proteins are extracellular epitopes of soluble or membrane bound transport proteins. In particular those epitopes accessible at the membrane binding site are preferred.Besides the transporter binding site of the modular antibody as engineered according to the invention further binding capacities may be present, constructed or introduced aside from or directly into the structural loop regions of constant or variable domains, e.g. binding capacities for other antigens, small molecules, for drugs or enzymes, catalytic sites of enzymes or enzyme substrates or the binding to a transition state analogue of an enzyme substrate.

Preferably the new transporter binding site of the modular antibody according to the invention is foreign to the modular antibody, i.e. the antibody did not contain a binding site to the transporter within the structural loops or CDR loops, or within the antibody domain, or even within the whole molecule. In particular embodiments targets foreign to the Fc fragment of an IgG antibody, like effector molecules, serum proteins or Fc-receptors or cell surface receptors, excluding those that are naturally bound to the CH2 and CH3 antibody domains, may be preferred as transporter molecules bound by the new binding site.

As used herein, the term "specifically binds" or "specific binding" refers to a binding reaction which is determinative of the cognate ligand of interest in a heterogeneous population of molecules. Thus, under designated conditions (e.g. immunoassay conditions), the modular antibody binds to its particular "target" and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10 fold different, preferably the difference is at least 100 fold, more preferred a least 1000 fold.

The term "expression system" refers to nucleic acid molecules containing a desired coding sequence and control sequences in operable linkage, so that hosts transformed or transfected with these sequences are capable of producing the encoded proteins. In order to effect transformation, the expression system may be included on a vector; however, the relevant DNA may then also be integrated into the host chromosome. Alternatively, an expression system can be used for in vitro transcription/translation.

A "structural loop" or "non-CDR-loop" according to the present invention is to be understood in the following manner: modular antibodies or immunoglobulins are made of domains with a so called immunoglobulin fold. In essence, anti-parallel beta sheets are connected by loops to form a compressed antiparallel beta barrel. In the variable region, some of the loops of the domains contribute essentially to the specificity of the antibody, i.e. the binding to an antigen. These loops are called CDR-loops. All other loops of antibody variable domains are rather contributing to the structure of the molecule and/or interaction with other domains. These loops are defined herein as structural loops or non-CDR-loops.

All numbering of the amino acid sequences of the immunoglobulins is according to the IMGT numbering scheme (IMGT, the international ImMunoGeneTics, Lefranc et al., 1999, Nucleic Acids Res. 27: 209-212; Ruiz et al., 2000 Nucleic Acids Res. 28: 219-221; Lefranc et al., 2001, Nucleic Acids Res. 29: 207-209; Lefranc et al., 2003, Nucleic Acids Res. 31: 307-310; Lefranc et al., 2005, Dev Comp Immunol 29:185-203).

According to a preferred embodiment of the present invention the modular antibody is of human, camelid, rabbit, chicken, rat, dog, horse, sheep or murine origin.

Since the modular antibody according to the invention may be employed for various purposes, in particular in pharmaceutical compositions, the modular antibody is preferably of human, camelid or murine origin. Of course, the modular antibody may also be a humanized or a chimeric immunoglobulin. In the most preferred embodiment of the invention the modular antibody is of human origin or a humanized version of a variable domain of any species.

A humanized modular antibody has at least about 50% amino acid sequence identity, preferably at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native human modular antibody sequence.

A humanized modular antibody has furthermore at least about 50% amino acid sequence identity, preferably at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity when comparing all surface accessible amino acids to the surface accessible amino acids of a native human modular antibody sequence.

The preferred homology or sequence identities specifically relates to those sequences of the framework region.

The preferred modular antibody according to the invention is selected from the group of VH, VL, including Vkappa and Vlambda, VHH, dAb, and combinations thereof. It turned out that those modifications are advantageous when brought into the loop regions of a VH, a Vkappa, a Vlambda or a VHH, and the modified loop regions comprise at least one modification within amino acids 7 to 21, amino acids 25 to 39, amino acids 41 to 81, amino acids 83 to 85, amino acids 89 to 103 or amino acids 106 to 117.

The structural loop regions of the modular antibody or immunoglobulin of human or humanized origin as modified according to the invention are selected preferably from the structural loops that comprise amino acids 8 to 20, amino acids 44 to 50, amino acids 67 to 76 and amino acids 89 to 101, most preferably amino acid positions 12 to 17, amino acid positions 45 to 50, amino acid positions 69 to 75 and amino acid positions 93 to 98.

In another preferred embodiment a modification in the structural loop region comprising amino acids 93 to 98 is combined with a modification in the structural loop region comprising amino acids 8 to 20.

The above identified amino acid regions of the respective immunoglobulins comprise loop regions to be modified. Preferably, a modification in the structural loop region comprising amino acids 93 to 98 is combined with a modification in one or more of the other structural loops.

In a preferred embodiment a modification in the structural loop region comprising amino acids 93 to 98 is combined with a modification in the structural loop region comprising amino acids 69 to 75.

Most preferably each of the structural loops comprising amino acids 93 to 98, amino acids 69 to 75 and amino acids 8 to 20 contain at least one amino acid modification.

In another preferred embodiment each of the structural loops comprising amino acids 93 to 98, amino acids 69 to 75, amino acids 44 to 50 and amino acids 8 to 20 contain at least one amino acid modification.

According to a preferred embodiment of the present invention the structural loop regions of a modular antibody or an immunoglobulin of murine origin are modified, e.g. within a VH, in the region of amino acids 6 to 20, amino acids 44 to 52, amino acids 67 to 76 and amino acids 92 to 101.

According to another preferred embodiment of the present invention the structural loop regions of a modular antibody or an immunoglobulins of camelid origin are modified, e.g. within a VHH, in the region of amino acids 7 to 18, amino acids 43 to 55, amino acids 68 to 75 and amino acids 91 to 101.

The variable domains of camelid origin or humanized variants of camelid origin, have the advantage that they could easily be combined with other variable domains, for instance with other VHH of camelid origin, modified or native. The possible combination of VHH of camelid origin is the basis for multivalent immunoglobulins. Thus, according to the invention specific modified variable domains of camelid origin are multivalent combinations, preferably with at least 3, more preferably with at least 4 or 5 valencies or VHHs.

In general the modular antibody according to the invention may be used as a building block to molecularly combine other modular antibodies or biologically active substances or molecules. It is preferred to combine molecularly at least one antibody binding to the specific partner via the variable or non-variable sequences, like structural loops, with at least one other binding molecule which can be an antibody, antibody fragment, a soluble receptor, a ligand or another antibody domain. Other combinations refer to proteinaceous molecules, nucleic acids, lipids, organic molecules and carbohydrates.

According to another preferred embodiment of the present invention the modification of at least one nucleotide in each of at least two structural loop regions results in a substitution, deletion and/or insertion in the modular antibody or immunoglobulin encoded by said nucleic acid.

Preferably, the new transporter binding sites in the structural loops are introduced in the modular antibody encoding the selected nucleic acid by substitution, deletion and/or insertion of at least one nucleotide.

The modification of modular antibody may result in a substitution, deletion and/or insertion of 2 or more amino acids, preferably point mutations, change of amino acids of whole loops, more preferred the change of at least 2, 3, 4, 5, 6, 7, 8, 9, 10, 11, 12, 13, 14, up to 30 amino acids. However, the maximum number of amino acids inserted into a structural loop region of an immunoglobulin or immunoglobulin variable domain may in specific cases not exceed the number of 30, preferably 25, more preferably 20, amino acids, to maintain the native structure of the modular antibody.

A preferred method to introduce modifications is site directed random mutation. With this method one, two or more specific amino acid residues of the loops are exchanged or introduced using randomly generated inserts into such structural loops. Alternatively preferred is the use of combinatorial approaches.

The modular antibody loop regions are preferably mutated or modified by random, semi-random or, in particular, by site-directed random mutagenesis methods. It turned out that randomization of at least two, or even three or more structural loops are most preferred to create the novel transporter binding site.

These randomization methods may be used to make amino acid modifications at desired positions of the modular antibody or immunoglobulins of the present invention. In these cases positions are chosen randomly, or amino acid changes are made using certain rules. For example certain residues may be mutated to any amino acids, whereas other residues may be mutated to a restricted set of amino acids. This can be achieved in a stepwise fashion by alternating of cycles of mutation and selection or simultaneously.

A preferred method according to the invention refers to a randomly modified nucleic acid molecule coding for a modular antibody or a part thereof which comprises at least one nucleotide repeating unit within a structural loop coding region having the sequence 5'-NNS-3', 5'-NNN-3'or 5'- NNK-3'. In some embodiments the modified nucleic acid comprises nucleotide codons selected from the group of TMT, WMT, RMC, RMG, MRT, SRC, KMT, RST, YMT, MKC, RSA, RRC, NNK, NNN,NNS or any combination thereof (the coding is according to IUPAC).

Enrichment with certain amino acids such as basic amino acids (e.g. Histidine) can be achieved by changing a natural amino acid at a certain position against Histidine. Any position on the protein can thereby be changed to Histidine. This method works analogous to the well-known Alanine scanning technique and is referred hereto as Histidine scanning. The technique can be used to change two or even three residues simultaneously.

Enrichment of library members with a certain amino acid, e.g histidine, may be achieved by doping the library-forming oligonucleotides with a certain percentage, e.g. 1%, preferably 10%, more preferably 20% most preferably 80%, of oligonucleotides encoding the respective amino acid. For example, doping with CAT, CAC or CAY codons at various positions in library gene synthesis allows for enrichment of Histidine containing library members.

The randomly modified nucleic acid molecule may comprise the above identified repeating units, which code for all known naturally occurring amino acids or a subset thereof.

The modification of the nucleic acid molecule may be performed by introducing synthetic oligonuleotides into a larger segment of nucleic acid or by de novo synthesis of a complete nucleic acid molecule. Synthesis of nucleic acid may be performed with tri-nucleotide building blocks which would reduce the number of nonsense sequence combinations if a subset of amino acids is to be encoded (e.g. Yanez et al. Nucleic Acids Res. (2004) 32:e158; Virnekas et al. Nucleic Acids Res. (1994) 22:5600-5607).

Preferably the positions to be modified are surface exposed amino acids. Surface exposition of amino acids of structural loops can be judged from known protein structures of modular antibodies and by analogy or homology for such amino acid sequences for which no experimentally determined structure is available.

In a preferred embodiment of the invention the modifications introduced into the modular antibody, in particular in the structural loops comprise at least 1, 2, 3, 4, 5, 6 foreign amino acids or amino acids not naturally occurring at the respective site of the structural loop of the non-modified immunoglobulin or immunoglobulin variable domain.

pH dependency of binding to the transport protein is a key selection parameter to obtain those molecules that have proven to cross epithelial barriers and to utilize the human salvage system of protecting molecules from early proteolytic attack and degradation. Though libraries have been described by WO 06072620A1, which libraries include immunogobulins in the native form, it was not expected that specific randomization regarding only minor changes in the structural loops could result in pH dependent binders of transport proteins. A few amino acid changes, i.e. substitutions, deletions and/or insertions, were surprisingly found to make a huge difference in the pH dependency of binding of novel binding sites in the structural loop regions. Thus, modular antibody binders of transport proteins are provided in the native form, preferably by modifications in the structural loops without destruction of the native, correct overall folding of the immunoglobulin domain and consequently also to the correct positioning of any existing CDRs and to interaction between domains, followed by selection of specific pH dependent binding properties.

Preferred properties of the pH dependent binding site are the binding of the transport protein, such as FcRn, at pH 5-7 with higher affinity than at pH 7.1-7.6, in particular with at least 10 fold, preferably at least 100 fold, more preferred at least 1000 fold affinity at the acidic pH. The pH dependent binding site according to the invention, as determined by its amino acid sequence and tertiary structure of the binding site within the protein scaffold, enables binding of the modular antibody to a transport protein with high affinity at pH 6 with a Kd in the range of 10⁻⁹ to 10⁻¹². It may however sometimes be desirable to obtain a binder with moderate binding affinity of 10⁻⁷ to 10⁻⁸. The most preferred binders are however engineered to bind FcRn with nanomolar affinity at pH 5-6, but showing no detectable binding at pH 7.5, meaning less than 5% of binding at pH 6, preferably less than 1%.

The pH dependency of binding transport proteins was preferably obtained with modifications introducing amino acids with side chain acidity or basicity, basic amino acids being most preferred, among them Arginine, Histidine and Lysine. Specially designed libraries are produced by randomization through substitution or insertion of at least one of the amino acids with side chain acidity or basicity, preferably at least two of those amino acids in proximal distance. These preferred libraries are also called focussed libraries.

"Proximal distance" is preferably defined within 10 amino acid positions, or even closer, within 8 or 5 amino acids. Most preferred are libraries with accumulated basic amino acids in the structural loop region or within the framework region, or most preferred within the binding site spanning over at least 2 loops of a modular antibody scaffold. Thereby the chances of successful panning and selecting molecules that bind transporter proteins in a pH dependent manner are highly increased.

Specific Histidine-enriched libraries of modular antibodies, in particular antibody fragments, such as Fab, Fc, or domain antibodies, are preferred. Thus, according to the invention it is the first time possible to provide libraries of modular antibodies comprising CH1/CL domains or variable domains, containing at least one Histidine or other basic amino acid, preferably in a structural loop position, preferably at the C-terminal loops, more preferred in at least two of the C-terminal loops or at least two Histidine residues within one C-terminal loop. Further preferred embodiments are libraries of modular antibodies with randomized C-terminal loops of CH3 domains. Those libraries preferably contain the basic amino acids, like histidine. According to the invention it is thus the first time possible to provide a Histidine-enriched library of modular antibodies containing randomized C-terminal loops of a CH3, preferably containing Histidine in at least two C-terminal loops of a modular antibody or containing at least two Histidine residues within one loop. The most preferred method to produce such binders is through design of dedicated libraries, employing the substitution and/or insertion of amino acids to introduce such amino acids with side chain acidity or basicity, e.g. Fab libraries, CH1/CL libraries and dAb libraries with enriched basic amino acids in loop positions.

Those libraries according to the invention, which are enriched in basic amino acids within one or more structural loops, specifically the Histidine enriched libraries, contain basic amino acid or Histidine enriched library members, i.e. those modular antibodies that contain either at least one of the basic amino acids within one structural loop, preferably at least two of the basic amino acids, or at least one of the basic amino acids in at least two of the structural loops, preferably at least two within a structural loop region, more preferred at least two within two loops, more preferred at least three or even at least four within two loops. The preferred dedicated library containing the basic amino acid enriched library members comprise a significant portion of such basic amino acid enriched library members, preferably at least 10%, more preferred at least 20%, more preferred at least 30%, more preferred at least 40%, more preferred at least 50%, more preferred at least 60%, more preferred at least 70%, more preferred at least 80%, more preferred at least 90%, more preferred at least 95%, up to 99%.

The preferred libraries according to the invention are designed in the way that members of the library contain the novel transporter binding site, which does not interfere with an optional natural FcRn binding site of an antibody. Thus, the additional binding of a transport protein through the novel pH dependent binding site may even prolong the half-life of an antibody to longer than the natural one. By the method according to the invention antibodies may be produced that exert an in vivo half-life of longer than 20 days, even longer than 30 days.

In one preferred embodiment, a library of polypeptide variants comprising modular antibodies according to the invention is used as a pool for selection wherein the modifications contain or introduce at least one, more preferably at least two amino acids per modified structural loop out of the group of amino acids tryptophane, tyrosine, phenylalanine, histidine, isoleucine, serine, methionine, alanine and asparagine.

Further preferred specific mutations result in the introduction of other foreign amino acids to the native polypeptides. Any of the amino acids tryptophane, tyrosine, phenylalanine, histidine, isoleucine, serine, methionine, alanine and asparagines are not present in the structural loops of human native immunoglobulins, thus are considered as "foreign". A variant modular antibody according to the invention may contain at least two of said foreign amino acids within the framework region, preferably in the structural loops, to make up a binding site, preferably in proximal distance.

If the modified modular antibody is of human origin or a humanized immunoglobulin variable domain preferred modifications are the incorporation of least one tyrosine in any one of the positions 12 to 17, 45 to 50, 69 to 75 and 93 to 98, and/or at least one tryptophane in any one of the positions 12 to 17, 45 to 50, 69, 71 to 75, 93 to 94 and 96 to 98, and/or at least one histidine in any one of the positions 12 to 17, 46, 47, 49, 50, 69 to 74 and 93 to 98, and/or at least one asparagine in any one of the positions 12 to 17, 45 to 47, 49, 50, 70 to 73, 75, 94 to 96 and 98, and/or at least one methionine in any one of the positions 12 to 17, 46 to 50, 69 to 71, 73 to 75, 93, 95, 96 and 98, and/or at least one serine in any one of the positions 13, 71, 75, 94, 95 and 98, and/or at least one isoleucine in any one of the positions 12, 14 to 17, 45 to 50,69, 70, 72 to 75, 93 and 96 to 98, and/or at least one phenylalanine in any one of the positions 15, 46, 48, 70 to 73, 75, 93, 95 and 98.

According to another preferred embodiment of the present invention at least two amino acid residues in positions 15 to 17, 29 to 34, 85.4 to 85.3, 92 to 94, 97 to 98 and/or 108 to 110 of a human or humanized single domain antibody are modified.

The modification of amino acids may preferentially be biased in order to introduce into structural loop regions amino acids which are known to be frequently involved in protein-protein interactions (e.g. Lea & Stewart (1995) FASEB J. 9:87-93; Fellhouse et al. (2006) J. Mol. Biol. 357:100-114; Adib-Conquuy et al. (1998) International Immunology 10:341-346; Lo Conte et al. (1999) J. Mol. Biol. 285:2177-2198; Zemlin et al. (2003) J. Mol. Biol. 334:733-749).

According to one embodiment of the invention a modular antibody obtainable by the method according to the invention is used again as a scaffold or as a combined protein scaffold for the preparation of a new protein library of modular antibodies.

The nucleic acid molecules encoding the modular antibody according to the invention (and always included throughout the whole specification: immunoglobulins and immunoglobulin fragments comprising a modular antibody) may be cloned into host cells, expressed and assayed for their binding specificities. These practices are carried out using well-known procedures, and a variety of methods that may find use in the present invention are described e.g. in Molecular Cloning--A Laboratory Manual, 3.sup.rd Ed. (Maniatis, Cold Spring Harbor Laboratory Press, New York, 2001), and Current Protocols in Molecular Biology (John Wiley & Sons). The nucleic acids that encode the modular antibodies of the present invention may be incorporated into an expression vector in order to express said immunoglobulins. Expression vectors typically comprise an immunoglobulin operably linked - that is placed in a functional relationship - with control or regulatory sequences, selectable markers, any fusion partners, and/or additional elements. The modular antibody of the present invention may be produced by culturing a host cell transformed with nucleic acid, preferably an expression vector, containing nucleic acid encoding the modular antibody, under the appropriate conditions to induce or cause expression of the modular antibody.

The methods of introducing exogenous nucleic acid molecules into a host are well known in the art, and will vary with the host used. Of course, also non-cellular or cell-free expression systems for the expression of immunoglobulins may be employed.

According to a preferred embodiment of the present invention the expression system comprises a vector. Any expression vector known in the art may be used for this purpose as appropriate.

The modular antibody is preferably expressed in a host, preferably in a bacterium, in yeast, in a plant cell, in an insect cell, in an animal cell or mammalian cell or in an organ of a plant or animal or in a complete plant or animal.

A wide variety of appropriate host cells may be used to express the polypeptides of the invention, including but not limited to mammalian cells (animal cells) plant cells, bacteria (e.g. Bacillus subtilis, Escherichia coli), insect cells, and yeast (e.g. Pichia pastoris, Saccharomyces cerevisiae). For example, a variety of cell lines that may find use in the present invention are described in the ATCC cell line catalog, available from the American Type Culture Collection. Furthermore, also plants and animals may be used as hosts for the expression of the immunoglobulin according to the present invention. The expression as well as the transfection vectors or cassettes may be selected according to the host used.

Of course also non-cellular or cell-free protein expression systems may be used. In vitro transcription/translation protein expression platforms, that produce sufficient amounts of protein offer many advantages of a cell-free protein expression, eliminating the need for laborious up- and down-stream steps (e.g. host cell transformation, culturing, or lysis) typically associated with cell-based expression systems.

In a preferred embodiment of the present invention, the modular antibodies are purified or isolated after expression modular antibodies may be isolated or purified in a variety of ways known to those skilled in the art. Standard purification methods include chromatographic techniques, electrophoretic, immunological, precipitation, dialysis, filtration, concentration, and chromatofocusing techniques. Purification can often be enabled by a particular fusion partner. For example, antibodys may be purified using glutathione resin if a GST fusion is employed, Ni2+-affinity chromatography if a His-tag is employed or immobilized anti-flag antibody if a flag-tag is used. For general guidance in suitable purification techniques, see e.g. Scopes, "Protein Purification: Principles and Practice", 1994, 3rd ed., Springer-Science and Business Media Inc., NY or Roe, "Protein Purification Techniques : A Practical Approach", 2001, Oxford University Press. Of course, it is also possible to express the modular antibodies according to the present invention on the surface of a host, in particular on the surface of a bacterial, insect or yeast cell or on the surface of phages or viruses.

Modified modular antibody of the invention may be screened using a variety of methods, including but not limited to those that use in vitro assays, in vivo and cell-based assays, and selection technologies. Automation and high-throughput screening technologies may be utilized in the screening procedures. Screening may employ the use of a fusion partner or label, for example an enzyme, an immune label, isotopic label, or small molecule label such as a fluorescent or colorimetric dye or a luminogenic molecule.

In a preferred embodiment, the functional and/or biophysical properties of the modular antibody are screened in an in vitro or ex vivo assay. In a preferred embodiment, the antibody is screened for functionality, for example its ability to catalyze a reaction or its binding specificity, cross-linking properties, cross reactivity and/or affinity to its target.

In another preferred embodiment, the favourable modular antibody may be selected in vivo, e.g. by introducing it into a cell or an organism. The specifically binding variants may be isolated either from body fluid such as blood or lymphatic liquid or from specific organs, depending on the required properties of the modified domains.

Assays may employ a variety of detection methods including but not limited to chromogenic, fluorescent, luminescent, or isotopic labels.

As is well known in the art, there are a variety of selection technologies that may be used for the identification and isolation of proteins with certain binding characteristics and affinities, including, for example, display technologies such as phage display, ribosome display, cell surface display, and the like, as described below. Methods for production and screening of antibody variants are well known in the art. General methods for antibody molecular biology, expression, purification, and screening are described e.g. in Antibody Engineering, edited by Duebel & Kontermann, Springer-Verlag, Heidelberg, 2001.
As is known in the art, some screening methods select for favourable members of a library. The methods are herein referred to as "selection methods", and these methods find use in the present invention for screening modular antibodies. When variant modular antibody libraries are screened using a selection method, only those members of a library that are favourable, that is which meet some selection criteria, are propagated, isolated, and/or observed. As will be appreciated, because only the fittest variants are observed, such methods enable the screening of libraries that are larger than those screenable by methods that assay the fitness of library members individually. Selection is enabled by any method, technique, or fusion partner that links, covalently or non-covalently, the phenotype of immunoglobulins with its genotype, that is the function of an antibody with the nucleic acid that encodes it. For example the use of phage display as a selection method is enabled by the fusion of library members to a phage coat protein at the N- or at the C-terminus or on both termini (most frequently used is the filamentous phage gene III protein, however also other coat proteins such as protein VIII, protein VII, protein VI and protein IX can be used). In this way, selection or isolation of modified immunoglobulins that meet some criteria, for example binding affinity to the immunoglobulin's target, also selects for or isolates the nucleic acid that encodes it. Once isolated, the gene or genes encoding modular antibodies or immunoglobulins may then be amplified. This process of isolation and amplification, referred to as panning, may be repeated, allowing favourable antibody variable domain variants in the library to be enriched. Nucleic acid sequencing of the attached nucleic acid ultimately allows for gene identification.

A variety of selection methods are known in the art that may find use in the present invention for screening modular antibody libraries. These include but are not limited to phage display (see e.g. Phage display of peptides and antibodies: a laboratory manual, Kay et al., 1996, Academic Press, San Diego, Calif., 1996) and its derivatives such as selective phage infection (Malmborg et al., 1997, J Mol Biol 273:544-551), selectively infective phage (Krebber et al., 1997, J Mol Biol 268:619-630), and delayed infectivity panning (Benhar et al., 2000, J Mol Biol 301:893-904), cell surface display (Witrrup, 2001, Curr Opin Biotechnol, 12:395-399) such as display on bacteria (Georgiou et al., 1997, Nat Biotechnol 15:29-34; Georgiou et al., 1993, Trends Biotechnol 11:6-10; Lee et al., 2000, Nat Biotechnol 18:645-648; Jun et al., 1998, Nat Biotechnol 16:576-80), yeast (Boder & Wittrup, 2000, Methods Enzymol 328:430-44; Boder & Wittrup, 1997, Nat Biotechnol 15:553-557), and mammalian cells (Whitehom et al., 1995, Bio/technology 13:1215-1219), as well as in vitro display technologies (Amstutz et al., 2001, Curr Opin Biotechnol 12:400-405) such as polysome display (Mattheakis et al., 1994, Proc Natl Acad Sci USA 91:9022-9026), ribosome display (Hanes et al., 1997, Proc Natl Acad Sci USA 94:4937-4942), mRNA display (Roberts & Szostak, 1997, Proc Natl Acad Sci USA 94:12297-12302; Nemoto et al., 1997, FEBS Lett 414:405-408), and ribosome-inactivation display system (Zhou et al., 2002, J Am Chem Soc 124, 538-543).

Other selection methods that may find use in the present invention include methods that do not rely on display, such as in vivo methods including but not limited to periplasmic expression and cytometric screening (Chen et al., 2001, Nat Biotechnol 19:537-542), the antibody fragment complementation assay (Johnsson & Varshavsky, 1994, Proc Natl Acad Sci USA 91:10340-10344; Pelletier et al., 1998, Proc Natl Acad Sci USA 95:12141-12146), and the yeast two hybrid screen (Fields & Song, 1989, Nature 340:245-246) used in selection mode (Visintin et al., 1999, Proc Natl Acad Sci USA 96:11723-11728). In an alternate embodiment, selection is enabled by a fusion partner that binds to a specific sequence on the expression vector, thus linking covalently or noncovalently the fusion partner and associated immunoglobulin library member with the nucleic acid that encodes them. For example, WO9308278 describe such a fusion partner and technique that may find use in the present invention. In an alternative embodiment, in vivo selection can occur if expression of the antibody imparts some growth, reproduction, or survival advantage to the cell.

Some selection methods are referred to as "directed evolution" methods. Those methods include the mating or breeding of favourable sequences during selection, sometimes with the incorporation of new mutations. As will be appreciated by those skilled in the art, directed evolution methods can facilitate identification of the most favourable sequences in a plurality of polypeptides, and can increase the diversity of sequences that are screened. A variety of directed evolution methods are known in the art that may find use in the present invention for generating and screening antibody variable domain variants, including but not limited to DNA shuffling (PCT WO00/42561; PCT WO 01/70947), exon shuffling (Kolkman & Stemmer, 2001, Nat Biotechnol 19:423-428), family shuffling (Crameri et al., 1998, Nature 391:288-291), selective combinatorial randomization (WO0301210 WO04018674A1), Random Chimeragenesis on Transient Templates (Coco et al., 2001, Nat Biotechnol 19:354-359), molecular evolution by staggered extension process (StEP) in vitro recombination (Zhao et al., 1998, Nat Biotechnol 16:258-261; Shao et al., 1998, Nucleic Acids Res 26:681-683), exonuclease mediated gene assembly (U.S. Pat. No. 6,352,842; U.S. Pat. No. 6,361,974), Gene Site Saturation Mutagenesis (U.S. Pat. No. 6,358,709), Gene Reassembly (U.S. Pat. No. 6,358,709), SCRATCHY (Lutz et al., 2001, Proc Natl Acad Sci USA 98:11248-11253), DNA fragmentation methods (Kikuchi et al., Gene 236:159-167), single-stranded DNA shuffling (Kikuchi et al., 2000, Gene 243:133-137), and directed evolution antibody engineering technology (Applied Molecular Evolution) (U.S. Pat. No. 5,824,514; U.S. Pat. No. 5,817,483; U.S. Pat. No. 5,814,476; U.S. Pat. No. 5,763,192; U.S. Pat. No. 5,723,323).

In a preferred embodiment, modular antibody variants are screened using one or more cell-based or in vivo assays. For such assays, purified or non-purified modular antibodies are typically added exogenously such that cells are exposed to modular antibodies or pools of modular antibody variants belonging to a library. These assays are typically, but not always, based on the desired function of modular antibodies; that is, the ability of the antibody or antibody domains as provided according to the invention to bind to its target and to mediate some biochemical event, for example effector function, ligand/receptor binding inhibition, apoptosis, and the like. Such assays often involve monitoring the response of cells to the modular antibody, for example cell survival, cell death, change in cellular morphology, or transcriptional activation such as cellular expression of a natural gene or reporter gene. For example, such assays may measure the ability of modular antibody variants to elicit ADCC, ADCP or CDC. For some assays additional cells or components, that is in addition to the target cells, may need to be added, for example serum complement, or effector cells such as peripheral blood monocytes (PBMCs), NK cells, macrophages, and the like. Such additional cells may be from any organism, preferably humans, mice, rat, rabbit, and monkey. Immunoglobulins may cause apoptosis of certain cell lines expressing the target, or they may mediate attack on target cells by immune cells which have been added to the assay. Methods for monitoring cell death or viability are known in the art, and include the use of dyes, immunochemical, cytochemical, and radioactive reagents. For example, caspase staining assays may enable to measure apoptosis, and uptake or release of radioactive substrates or fluorescent dyes may enable cell growth or activation to be monitored.

Alternatively, dead or damaged target cells may be monitored by measuring the release of one or more natural intracellular components, e.g. lactate dehydrogenase. Transcriptional activation may also serve as a method for assaying function in cell-based assays. In this case, response may be monitored by assaying for natural genes which may be upregulated, for example the release of certain interleukins may be measured, or alternatively readout may be via a reporter system. Cell-based assays may also involve the measure of morphological changes of cells as a response to the presence of modular antibody variants. Cell types for such assays may be prokaryotic or eukaryotic, and a variety of cell lines that are known in the art may be employed.

Alternatively, cell-based screens may be performed using cells that have been transformed or transfected with nucleic acids encoding the variant modular antibodies. In this case, modular antibody variants of the invention are not added exogenously to the cells (e.g. Auf der Maur, 2004, Methods, 34:215-224). In another alternative method, the cell-based screen utilizes cell surface display. A fusion partner can be employed that enables display of modified immunoglobulin variable domains on the surface of cells (Witrrup, 2001, Curr Opin Biotechnol, 12:395-399).

In a preferred embodiment the binder of the transport protein is selected or characterized by the specific ex vivo assay. Such assay employs a cell-layer that expresses the transport protein that could bind the modular antibody in a pH dependent way, internalize and pass the modular antibody through cell barriers.

There are several approaches for suitable cell layer assays to obtain a system which may afford the transcytosis of modular antibodies or phages, in particular those presenting modular antibodies, via the FcRn and which may therefore be suitable for a screening system:
(i) Several human choriocarcinoma cell lines are commercially available (e.g. JEG-3, BeWo or CaCo-2 cells).
(ii) There are human epithelial cell lines such as T84, which also support FcRn-mediated transcytosis of IgG (Dickinson et al . J Clin Invest. 1999, 104:903-11).
(iii) FcRn may be heterologously expressed in the dog kidney cell line MDCK (by stably transfecting these cells with plasmids encoding the alpha-chain of human FcRn and human beta2-microglobulin; see Claypool et al., J Biol Chem. 2002;277:28038-50; Tesar et al.; Traffic. 2006; 7:1127-42.

Each of these cell lines can be tested for their ability to support transcytosis of a phage that contains a well defined FcRn-binding motif.

Such an assay system can be employed to screen for sequences, which support tight binding to and cellular transcytosis by the FcRn such as screening for FcRn-binding sequences by phage display. In this regard a phage library, e.g. displaying modular antibodies of Fcab^{™} format, is screened to pass the respective cell layer, like monocell layers. Thereby the members of the library are screened for affinities and identified sequences that support efficient transcytosis. Trafficking of modular antibodies may be investigated in human endothelial cells. Endothelial cells continuously take up IgG via FcRn; these internalized antibodies are rapidly rerouted to the cell surface by recycling endosomes. This mechanism shields antibodies against degradation and accounts for the long half-life of IgG.

The biological or functional properties of the modular antibody of the present invention may be characterized in cell, tissue, and whole organism experiments. As is known in the art, drugs are often tested in animals, including but not limited to mice, rats, rabbits, dogs, cats, pigs, and monkeys, in order to measure a drug's efficacy for treatment against a disease or disease model, or to measure a drug's pharmacokinetics, toxicity, and other properties. The animals may be referred to as disease models. Therapeutics are often tested in mice, including but not limited to nude mice, SCID mice, xenograft mice, and transgenic mice (including genetic knock-in and knock-out mutants). Such experimentation may provide meaningful data for determination of the potential of the polypeptide variant to be used as a therapeutic. Any organism, preferably mammals, may be used for testing. Because of their genetic similarity to humans, monkeys can be suitable therapeutic models, and thus may be used to test the efficacy, toxicity, pharmacokinetics, or other property of the modular antibodies of the present invention. Testing drug candidates in humans are most frequently required for approval as therapeutics, and thus of course these experiments are contemplated. Thus the modular antibodies or antibody domains of the present invention may be tested in humans to determine their therapeutic efficacy, toxicity, immunogenicity, pharmacokinetics, and/or other clinical properties.

In a specific embodiment, the immunogenicity of the modified immunoglobulins may be changed and determined experimentally using one or more immunological or cell based assays (e.g. Koren et al., 2002, Current Pharmaceutical Biotechnology 3:349-360). In a preferred embodiment, ex vivo T-cell activation assays are used to experimentally quantitate immunogenicity. In this method, antigen-presenting cells and naive T-cells from matched donors are challenged with a peptide or whole antibody or immunoglobulin of interest one or more times. T-cell activation can be detected using a number of methods, e.g. by monitoring of cytokine release or measuring uptake of tritiated thymidine. In preferred embodiments, LUMINEX technology is used to measure cytokine release or interferon gamma production is monitored using ELISPOT assays.

Modular antibodies according to the invention as characterized by the native structure are preferably provided in a non-immunogenic form for passive immunotherapy. In the course of such passive immunotherapies antibodies can be administered frequently or as continuous infusion, without inducing undesired side reactions, such as the induction of neutralizing antibodies to a level that requires higher dosing, e.g. doubling of the dose, than normal.

In another preferred embodiment the modular antibody according to the invention is presented as an antigen. It may be advantageous that the parent modular antibody is designed as an antigen, such as used for active immunotherapy, like a vaccine. In this case the modular antibody according to the invention has been engineered to introduce the additional transcytotic property while maintaining the native, antigenic structure of the parent molecule, as determined by immune reactions in respective animal models.

Another feature of the native structure is determined by the stability of the molecule, either in solution, buffer or serum, or stability after administration. Modifications of the molecule would preferably not significantly change the stability of the molecule. Thus deterioration or destruction of its structure or conformation would be in principle avoided. A molecule would be considered significantly stable after modification, if its in vitro or ex vivo half-life in a buffer system or in human serum would not be significantly reduced, for instance by a factor of 10, preferably a factor of 5, even more preferred by a factor of 3 or 2. Though the modification according to the invention would in most cases lead to the increase of half-life in vivo mediated by the pH dependent binding of a transport protein, the preferred modular antibody would in addition proof its native structure through its stability in vitro or ex vivo, which is not overlaid by the effect of the binding to the transport protein.

If the parent antibody comprises binding sites through CDR loops or non-CDR loops, its native structure is preferably represented by the CDR or non-CDR loop conformation, which binding site is optionally still biologically active after modification, so to enable the stable binding of a target. While in a preferred embodiment one or more structural loops are modified to engineer the new pH dependent binding site to a transport protein, it is understood that the loop conformation of CDR or non-CDR loops is not significantly changed, so to keep the native, original binding property to other targets.

The molecular stability of a modular antibody may also be determined by its binding to Protein A or Protein G. It turned out that the natura binding site for Protein A, e.g. that is inherent to a native IgG, is an indicator for the molecular stability and epression stability of an antibody or an antibody Fc fragment, This is important for practical reasons, because the modular antibodies can be purified through affinity chromatography using those proteins as affinity ligands.

The biological or functional properties of the modular antibodies of the present invention may be characterized in cell, tissue, and whole organism experiments. As is known in the art, drugs are often tested in animals, including but not limited to mice, rats, rabbits, dogs, cats, pigs, and monkeys, in order to measure a drug's efficacy for treatment against a disease or disease model, or to measure a drug's pharmacokinetics, toxicity, and other properties. The animals may be referred to as disease models. Therapeutics are often tested in mice, including but not limited to nude mice, SCID mice, xenograft mice, and transgenic mice (including genetic knock-in and knock-out mutants). Such experimentation may provide meaningful data for determination of the potential of the polypeptide variant to be used as a therapeutic. Any organism, preferably mammals, may be used for testing. Because of their genetic similarity to humans, monkeys can be suitable therapeutic models, and thus may be used to test the efficacy, toxicity, pharmacokinetics, or other property of the modified immunoglobulins of the present invention. Testing drug candidates in humans are most frequently required for approval as therapeutics, and thus of course these experiments are contemplated. Thus the modular antibodies of the present invention may be tested in humans to determine their therapeutic efficacy, toxicity, immunogenicity, pharmacokinetics, and/or other clinical properties.

The modular antibodies of the present invention may find use in a wide range of antibody products. In one embodiment the modular antibodies or antibody variant of the present invention is used for therapy or prophylaxis, for preparative or analytic use, as a diagnostic, an industrial compound or a research reagent, preferably a therapeutic. The antibody variant may find use in an antibody composition that is monoclonal, oligoclonal or polyclonal. In a preferred embodiment, the modular antibodies of the present invention are used to kill target cells that bear the target antigen, for example cancer cells. In an alternate embodiment, the modular antibodies of the present invention are used to block, antagonize, or agonize the target antigen, for example by antagonizing a cytokine or a cytokine receptor thereof. In an alternately preferred embodiment, the modular antibodies of the present invention are used to block, antagonize, or agonize the target antigen and kill the target cells that bear the target antigen.

In an alternately preferred embodiment, the modular antibodies of the present invention are used to block, antagonize, or agonize growth factors or growth factor receptors and kill the target cells that bear or need the target antigen.

In an alternately preferred embodiment, the modular antibodies of the present invention are used to block, antagonize, or agonize enzymes and substrate of enzymes.

In another alternatively preferred embodiment, the modified immunoglobulin variable domains of the present invention are used to neutralize infectious agents such as viruses, small viruses, prions, bacteria or fungi.

The modular antibodies of the present invention may be used for various therapeutic purposes. In a preferred embodiment, an antibody comprising the modular antibodies or respective domains is administered to a patient to treat a specific disorder. A "patient" for the purposes of the present invention includes both, humans and other animals, preferably mammals and most preferably humans. By "specific disorder" herein is meant a disorder that may be ameliorated by the administration of a pharmaceutical composition comprising a modular antibodies of the present invention.

In one embodiment, a modular antibody according to the present invention is the only therapeutically active agent administered to a patient. Alternatively, the modular antibodies according the present invention is administered in combination with one or more other therapeutic agents, including but not limited to cytotoxic agents, chemotherapeutic agents, cytokines, growth inhibitory agents, anti-hormonal agents, kinase inhibitors, anti-angiogenic agents, cardioprotectants, or other therapeutic agents. The modular antibodies may be administered concomitantly with one or more other therapeutic regimens. For example, an antibody variant of the present invention may be formulated and administered to the patient along with chemotherapy, radiation therapy, or both chemotherapy and radiation therapy. In one embodiment, the modular antibodies of the present invention may be administered in conjunction with one or more antibodies, which may or may not comprise an antibody variant of the present invention. In accordance with another embodiment of the invention, the modular antibodies of the present invention and one or more other anti-cancer therapies are employed to treat cancer cells ex vivo. It is contemplated that such ex vivo treatment may be useful in bone marrow transplantation and particularly, autologous bone marrow transplantation. It is of course contemplated that the antibodies of the invention can be employed in combination with still other therapeutic techniques such as surgery.

A variety of other therapeutic agents may find use for administration with the modular antibodies of the present invention. In one embodiment, the modular antibody is administered with an anti-angiogenic agent, which is a compound that blocks, or interferes to some degree with the development of blood vessels. The anti-angiogenic factor may, for instance, be a small molecule or a protein, for example an antibody, Fc fusion, or cytokine, that binds to a growth factor or growth factor receptor involved in promoting angiogenesis. The preferred anti-angiogenic factor herein is an antibody that binds to Vascular Endothelial Growth Factor (VEGF). In an alternate embodiment, the modular antibody is administered with a therapeutic agent that induces or enhances adaptive immune response, for example an antibody that targets CTLA-4. In an alternate embodiment, the modified immunoglobulin is administered with a tyrosine kinase inhibitor, which is a molecule that inhibits to some extent tyrosine kinase activity of a tyrosine kinase. In an alternate embodiment, the modified immunoglobulins of the present invention are administered with a cytokine. By "cytokine" as used herein is meant a generic term for proteins released by one cell population that act on another cell as intercellular mediators including chemokines.

Pharmaceutical compositions are contemplated wherein modular antibodies of the present invention and one or more therapeutically active agents are formulated. Formulations are prepared for storage by mixing said modular antibodies having the desired degree of purity with optional pharmaceutically acceptable carriers, excipients or stabilizers (Remington's Pharmaceutical Sciences, 1980, 16th edition, Osol, A. Ed.,), in the form of lyophilized formulations or aqueous solutions. The formulations to be used for in vivo administration are preferably sterile. This is readily accomplished by filtration through sterile filtration membranes or other methods. The modified immunoglobulins and other therapeutically active agents disclosed herein may also be formulated as immunoliposomes, and/or entrapped in microcapsules.

Administration of the pharmaceutical composition comprising a modified immunoglobulin or immunoglobulin variable domain of the present invention, preferably in the form of a sterile aqueous solution, may be performed in a variety of ways, including, but not limited to, orally, sublingual, subcutaneously, intravenously, intranasally, intraotically, transdermally, topically, e.g. in the form of gels, salves, lotions, creams, etc., intraperitoneally, intramuscularly, intrapulmonary, vaginally, parenterally, rectally, intraocularly or by the mucosal route.

The transcytotic modular antibody according to the invention binds to the transport proteins in a natural way thus is able to pass epithelial or endothelial cells. Since binding in the endosome and subsequent dissociation at the cell surface is desirable, the molecule of the invention is designed to exhibit stronger binding to FcRn at pH 5-6, while maintaining similar or weaker binding at pH 7.4, for example as proven by the respective soluble FcRn or cell based assays. In this regards binding to the FcRn can be directly by the modular antibody according to the invention or mediated by another binder of FcRn that is bound by the modular antibody according to the invention by a pH dependent binding site. Examples for such mediated FcRn binding are those through binding of serum albumin, HAS, or immunoglobulins, IgG.

For instance, neonatal Fc-receptor is expressed on many cell surfaces, e.g. on the vascular endothelium, where it mediates endocytotic recycling of IgG and of albumin. Thus, IgG and albumin are taken up into the cells and rapidly recycled back to the cell surface via FcRn, which thereby shields IgG and albumin from lysosomal degradation. The receptor was originally discovered on trophoblast cells, i.e. epithelial lining of the placenta, where it mediates transcytosis of IgG and thus supplies the fetus with maternal antibodies. Many other epithelial cells, e.g. of the lung and intestines, also express FcRn. Thus, provided that therapeutically relevant proteins are designed to bind to FcRn, this receptor can be exploited to deliver molecules, such as modular antibodies, through a mucosal surface.

The invention is further described by means of the following Examples.

### Examples

### 1. Design of Fc mutant libraries

Libraries of Fc fragments for display on filamentous phage are designed and synthesized chemically (Geneart, Germany). The synthetic gene libraries are cloned into a phage display vector such as pHEN1 for surface display by protein III fusion.

The wild type Fc is randomized in the loops marked with bold letters. Amino acids are written in capital letters in the single letter code. The underlined amino acids are sequences modified to result in the desired libraries. The codons for the random sequences are written in small letters. There are two randomization strategies: In one type of library the codons used are of the nnk type in order to cover all 20 amino acids. In another type of library kmt codons are used to cover only amino acids frequently involved in ligand binding (serine, alanine, tyrosine and aspartic acid). Libraries are additionally synthesized in a histidine enriched mode (i.e. doping of the randomized amino acid positions with 10% triplets coding for histidine).

EPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLSL
SPGK

### Only the randomized loops are shown to characterize the respective libraries:

### Library "CH3+5 kmt"

**...RDEL-(kmt)₃-QVSL............L TV-(kmt)₈-RW-(kmt)₂-GNV...**

### Library "CH3+5 kmt" with GS -----SG,, stem" loop

**... RDEL-(kmt)₃-QVSL ..... LTV-GS-(kmt)₈-SG-RW-(kmt)₂ -GNV...**

### Library "CH3+5 kmt" with extended first loop

**...RDE-(kmt)₅-VSL.....LTV-(kmt)₈-RW-(kmt)₂-GNV...**

### Library "CH3+5 nnb"

**...RDEL-(nnb)₃-QVSL.....LTV-(nnb)₈-RW-(nnb)₂-GNV...**

### Library "CH3+5 nnb" with GS--------SG "stem" loop

**... RDEL-(nnb)₃-QVSL ..... LTV-GS-(nnb)₈-SG-RW-(nnb)₂-GNV...**

### Library "CH3+5 nnb" with extended first loop

**... RDE-kmt(nnb)₃kmt-VSL.....LTV-(nnb)₈-RW-(nnb)₂-GNV...**

The libraries are prepared according to standard procedures as described (e.g. Phage Display: A Practical Approach (The Practical Approach Series, 266) by Tim Clackson (Editor), Henry B. Lowman (Editor) Oxford University Press, 2004)

### 2. Selection of pH dependent binders to FcRn, IgG and serum albumin

Phages Libraries are resuspended in 5 ml 20 mM MES, pH 6.0/5% skimmed milk/ 0.05% Tween 20 and added (100 µl of 5×10¹²cfu/ml/well) to 20 wells of a Maxisorp immunoplate (Nunc, Rochester, NY) coated with 1µg of murine and human FcRn, human IgG and human serum albumin respectively and blocked with 5% skimmed milk. After incubation for 2 h at 37°C, wells are washed 10-30 times with 20 mM MES, pH 6.0/0.2% Tween 20/0.3 M NaCl and phage eluted by incubation in 100 µl PBS, pH 7.4/well for 30 min at 37°C. Phage are used to reinfect exponentially growing E. coli TG1 according to standard procedures. Three to six rounds of panning are conducted. 20 to 100 clones of the panning rounds are sequenced for the specific inserts.

### 3. Transcytosis assay

Fc mutants selected in example 2 are cloned into a eukaryotic episomal vector (pCEP4) and expressed in HEK-293 cells. The proteins are purified and are tested for transcytosis with monolayers of cells expressing functional FcRn (e.g. Studies are performed with selected Fc mutants alone or in the presence of human IgG or serum albumin.

T84 cells (passages 28-61) are grown on Transwell inserts (Corning-Costar Corp., Cambridge, Massachusetts, USA) as described by Dickinson et al; J. Clin. Invest. 104:903-911 (1999). Transcytosis experiments are performed in HBSS supplemented with 10 mM HEPES (pH 7.4) (HBSS+).

Purified human IgG (IgG) and purified Fc are biotinylated with sulfo-NHS-biotin. Protein concentrations are determined by the bicinchoninic acid method (Pierce Chemical Co.) and confirmed by SDS-PAGE and western blot. T84 cell monolayers exhibiting high electrical resistances (800-1,200 W/cm2) are equilibrated in HBSS+, and 60 nM biotin-labeled IgG or Fc mutant protein (6-600 nM) is added to the apical or basolateral reservoirs. As a nonspecific blocker, 0.5% Teleostean gelatin (Sigma Chemical Co.) is included with ligand. Unlabeled IgG or Fc (30 mM) or 0.1 mg/mL fragment B of Staphylococcal protein A (Pierce Chemical Co.) is being used as a competitive inhibitor in control experiments. When indicated, T84 cell monolayers are preincubated for 20 minutes with 0.1 mM bafilomycin A1 (Sigma Chemical Co.) to inhibit the vacuolar proton pump. Monolayers are incubated for 1 hour with ligand at either 37°C or 4°C, after which an aliquot of the contralateral well buffer is collected and 3-4 similarly treated Transwells are combined. Transported proteins are precipitated by acetone or trichloroacetic acid in the presence of 10 mg yeast tRNA and are analyzed by SDS-PAGE and ligand blot after reduction with DTT. In some studies, transported proteins are analyzed by ELISA.

## Claims

1. Transcytotic modular antibody containing a de novo pH dependent binding site for a transport protein.

2. Modular antibody according to claim 1, wherein the binding site has specificity for a transport protein that mediates endocytotic and/or transcytotic trafficking, preferably selected from the group consisting of FcRn, Albumin and IgG.

3. Modular antibody according to any of claims 1 or 2, which further contains a natural binding site against a transport protein.

4. Modular antibody according to any of claims 1 to 3, which further contains at least one binding site against one of FcRn and Protein A.

5. Modular antibody according to any of claims 1 to 4, wherein the binding site binds to the transport protein at pH 6-7 with higher affinity than at pH 7.1-7.6.

6. Modular antibody according to any of claims 1 to 5, which is composed of a member of the immunoglobulin superfamily, or proteins with immunoglobulin-like structure, or parts thereof.

7. Modular antibody according to claim 6, wherein the member of the immunoglobulin superfamily is selected from the group consisting of intact IgG, Fab, Fc, scFv and dAb.

8. Modular antibody according to claim 6, wherein the proteins with immunoglobulin-like structure are selected from the group consisting of fibronectin, TCR, transferrin and CTLA-4.

9. Modular antibody according to any of claims 1 to 8, which is composed of at least one protein domain in the native form, preferably an antibody domain.

10. Modular antibody according to any of claims 1 to 9, wherein the binding site is presented by at least a structural loop of a protein domain, preferably a constant or variable antibody domain.

11. Modular antibody according to any of claims 1 to 10, which is conjugated to biologically active structures, or fused therewith.

12. Modular antibody according to any of claims 1 to 11, wherein the binding site includes at least one of acidic or basic amino acids, preferably at least two of them, within sterical proximity.

13. Modular antibody according to any of claims 1 to 12, produced by engineering a molecule containing an antibody domain to modify a structural loop and determining the binding of said molecule to an epitope of a transport protein at a first pH, wherein the unmodified molecule does not significantly bind to said transport protein at a second pH, comprising the steps of:
- providing a nucleic acid encoding a molecule containing an antibody domain comprising at least one structural loop region,
- modifying at least one nucleotide residue of at least one of said structural loop regions,
- transferring said modified nucleic acid in an expression system,
- expressing said modified molecule,
- contacting the expressed modified molecule with an epitope of a transport protein, and
- determining whether said modified molecule binds to said epitope in a pH dependent manner.

14. Method of producing a modular antibody according to any of claims 1 to 12 by engineering a molecule containing an antibody domain to modify a structural loop and determining the binding of said molecule to an epitope of a transport protein at a first pH, wherein the unmodified molecule does not significantly bind to said transport protein at a second pH, comprising the steps of:
- providing a nucleic acid encoding a molecule containing an antibody domain comprising at least one structural loop region,
- modifying at least one nucleotide residue of at least one of said structural loop regions,
- transferring said modified nucleic acid in an expression system,
- expressing said modified molecule,
- contacting the expressed modified molecule with an epitope of a transport protein, and
- determining whether said modified molecule binds to said epitope in a pH dependent manner.

15. Method according to claim 14, wherein the nucleic acid is modified by one of randomization, nucleotide sequence grafting or fusion techniques.

16. Method of producing a modular antibody according to any of claims 1 to 12 by conjugating a peptide to a molecule containing an antibody domain, wherein the peptide is binding to a transport protein at a first pH, wherein the unmodified molecule does not significantly bind to said transport protein at a second pH.

17. Use of a modular antibody according to any of claims 1 to 12, for the preparation of a protein library.

18. Library comprising at least 10 modular antibodies according to any of claims 1 to 12.

19. Library according to claim 18 **characterized in that** it contains modular antibodies with mutations to obtain at least one acidic or basic amino acid in a structural loop region.

20. Library according to any of claims 18 to 19, **characterized in that** it contains modular antibodies with at least 2 proximal acidic or basic amino acids.

21. Library according to claim 20, **characterized in that** it contains modular antibodies with at least two structural loops with at least one acidic or basic amino acids in the at least two structural loops.

22. Library according to any of the claims 18 to 21, **characterized in that** it is selected from the group consisting of a VH library, VHH library, Vkappa library, Vlambda library, Fab library, a CH1/CL library and a CH3 library.

23. Library according to any of the claims 18 to 21, **characterized in that** it is selected from the group consisting of an IgG library, Fc library and a T-cell receptor library.

24. Library according to any of the claims 18 to 23, containing a portion of its members with basic amino acids in structural loops, which portion is higher than 10%.

25. Fc library according to claim 24, containing a portion of its members with binding properties to Protein A, which portion is higher than 10%.

26. Method for specifically isolating a transcytotic modular antibody binding to a transport protein from a library containing modular antibodies according to any one of the claims 1 to 12, comprising the steps of:
c. contacting said library with a test sample containing said transport protein at an acidic pH, isolating the specific antibody/transport protein complex formed by the binders to said transport protein from the non-binders, and
d. treating said specific antibody/transport protein complex at a neutral to basic pH to release the pH dependent binders.

27. Kit of binding partners containing
e. a library containing modular antibodies according to any one of the claims 1 to 12, and
f. a transport protein.

28. Use of a transport protein of a kit according to claim 27 for selecting a modular antibody from a library according to any one of claims 18 to 25.

29. Method of prolonging the half-life of a modular antibody by engineering a de novo pH dependent binding site for a transport protein into the modular antibody.

30. Method of prolonging half-life of a biologically active molecule, by conjugating with or fusing to a modular antibody according to any of the claims 1 to 12.

31. Method of producing a pharmaceutical preparation containing a transcytotic modular antibody according to any of the claims 1 to 12, for the immunotherapy of a patient.

32. Method according to claim 31, for the treatment of a patient by mucosal administration.
